# EUROPÄISCHE PATENTSCHRIFT

(11) **EP 0 567 469 B1**
(45) Veröffentlichungstag und Bekanntmachung des Hinweises auf die Patenterteilung: **27.08.1997**
(21) Anmeldenummer: 92901304.3
(22) Anmeldetag: 21.12.1991
(51) Int. Cl.: C07J 41/00, A61K 31/565, A61K 31/58, C07J 1/00, C07J 17/00, C07J 43/00, C07J 9/00, C07J 33/00

(54) **14beta-H-, 14- UND 15-EN-11beta-ARYL-4-ESTRENE**
14beta-H-, 14- AND 15-EN-11beta-ARYL-4-ESTRENES
14beta-H-, 14- ET 15-EN-11beta-ARYL-4-ESTRENES

(30) Priorität: 22.12.1990 DE 4042004
(43) Veröffentlichungstag der Anmeldung: 03.11.1993
(73) Patentinhaber: SCHERING AKTIENGESELLSCHAFT, 13353 Berlin (DE)
(72) Erfinder: SCHWEDE, Wolfgang, D-1000 Berlin 10 (DE); OTTOW, Eckhard, D-1000 Berlin 45 (DE); NEEF, Günter, D-1000 Berlin 42 (DE); CLEVE, Arwed, D-1000 Berlin 42 (DE); CHWALISZ, Krzysztof, D-1000 Berlin 45 (DE); MICHNA, Horst, D-1000 Berlin 22 (DE); FUHRMANN, Ulrike, D-1000 Berlin 15 (DE)
(86) Internationale Anmeldenummer: EP9102494
(87) Internationale Veröffentlichungsnummer: WO9211278

(56) Entgegenhaltungen:
- EP-A- 0 299 913
- EP-A- 0 360 369

## Beschreibung

Die vorliegende Erfindung betrifft Verbindungen der allgemeinen Formel I 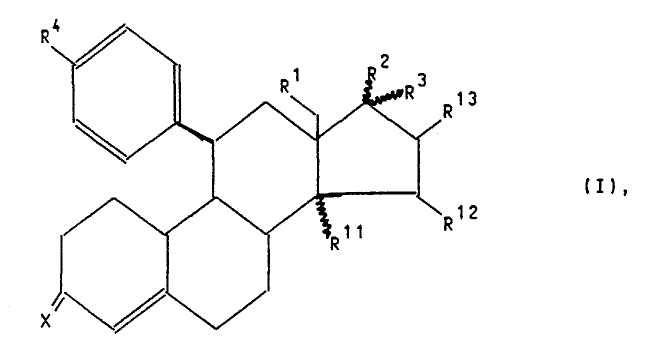 worin entweder
Ia) R¹¹ ein β-ständiges Wasserstoffatom sowie R¹² und R¹³ je ein Wasserstoffatom oder
Ib) R¹¹ ein β-ständiges Wasserstoffatom sowie R¹² und R¹³ gemeinsam eine zweite Bindung oder
Ic) R¹¹ und R¹² gemeinsam eine zweite Bindung sowie R¹³ ein Wasserstoffatom oder
Id) R¹¹ ein α-ständiges Wasserstoffatom sowie R¹² und R¹³ gemeinsam eine zweite Bindung
bedeuten sowie in Ia), Ib), Ic) oder Id)
- X: für ein Sauerstoffatom, die Hydroxyiminogruppierung >N~OH oder zwei Wasserstoffatome,
- R¹: für ein Wasserstoffatom oder eine Methylgruppe,
- R²: für eine Hydroxygruppe, eine C₁-C₁₀-Alkoxy- oder C₁-C₁₀-Acyloxygruppe,
- R³: für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest -OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor-Brom-oder Jod-Atom, einen C₁-C₁₀-Hydroxyalkyl-, C₁-C₁₀-Alkoxyalkyl-, C₁-C₁₀-Acyloxyalkylrest oder wenn m = 0 ist, eine Methylgruppe bedeuten, ferner die Gruppierung -(CH₂)ₚ-CH=CH-(CH₂)ₖCH₂R⁶, wobei p 0 oder 1 und k 0, 1 oder 2 und R⁶ ein Wasserstoffatom, eine Hydroxygruppe, einen C₁-C₄-Alkoxy- oder C₁-C₄-Acyloxyrest bedeuten,
wobei
in Ia) und Ib) R² α- und R³ β-ständig und
in Ic) und Id) R² β- und R³ α-ständig ist,
oder aber R² und R³ gemeinsam für einen Rest der Formel 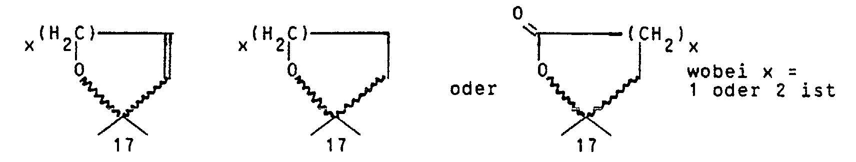 R⁴ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten C₁-C₈-Alkyl-, -Acyl-oder Alkoxyalkylrest, für
eine 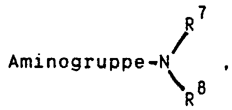 in welcher R⁷ und R⁸ unabhängig voneinander ein Wasserstoffatom oder eine C₁-C₄-Alkylgruppe bedeuten, oder für ein entsprechendes Aminoxid 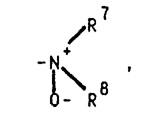 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα 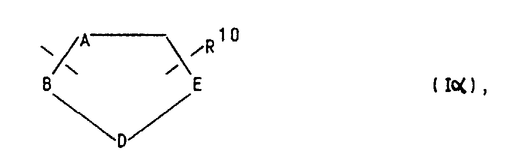 in welchem A ein Stickstoff-, Sauerstoff- oder Schwefelatom, -B-D-E- die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl-oder Alkoxyalkylrest, für
eine 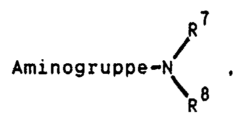 in welcher R⁷ und R⁸ unabhängig voneinander ein Wasserstoffatom oder eine C₁-C₄-Alkylgruppe bedeuten, oder ein entsprechendes Aminoxid 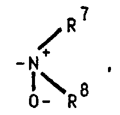 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ 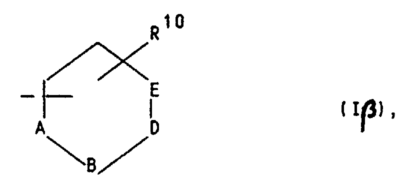 in welchem A ein Stickstoffatom und -B-D-E- die Elementenfolge -C-C-C-, -N-C-C-, -C-N-C- oder -C-C-N- bedeuten und R¹⁰ die bereits angegebene Bedeutung hat,
oder für einen Phenylrest der Formel Iγ 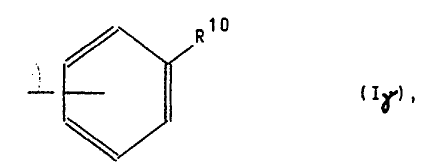 worin R¹⁰ die bereits angegebene Bedeutung hat,
stehen,
sowie deren pharmakologisch verträgliche Additionssalze mit Säuren, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die Erfindung betrifft insbesondere Verbindungen, in denen X für ein Sauerstoffatom steht.

Die in R², R³, R⁵ und Y der allgemeinen Formel I enthaltenen Alkoxy-, Acyloxy-, Alkyl-, Acyl- sowie Hydroxyalkylgruppen sollen jeweils 1 bis 10 und die Alkoxyalkyl- oder Acyloxyalkylgruppen in Y 2 bis 10 Kohlenstoffatome enthalten. Dabei sind als bevorzugte Gruppen von den Alkoxygruppen die Methoxy-, Ethoxy-, Propoxy- und Isopropoxygruppe zu nennen, von den Acyl(oxy)gruppen kommt der Formyl(oxy)-, Acetyl(oxy)- und Propionyl(oxy)gruppe besondere Bedeutung zu.

Bei den Alkylgruppen sind vor allem die Methyl-, Ethyl-, Propyl-, Isopropyl-sowie tert.-Butylgruppe zu nennen und von den Hydroxyalkylgruppen sind die entsprechenden, in beliebiger Stellung mit einer Hydroxygruppe substituierten Reste bevorzugt.

Für n kommt insbesondere 0, 1, 2 und 3 infrage; wenn Z=CN, ist eine Cyanomethyl gruppe (n=0) besonders bevorzugt. Außer den bereits genannten Gruppen kann Y vorzugsweise auch ein Wasserstoff-, Chlor- oder Brom-Atom sein.

Von den Alkenylresten in R³ sind die Propenyl- und Butenylgruppen, die in der E- oder Z-Konfigurationm vorliegen können, bevorzugt, das heißt, wenn R³ für -(CH₂)ₚ-CH=CH-(CH₂)ₖ-CH₂-R⁶ steht, dann soll k vorzugsweise 0 oder 1 und p=0 sein.
Unter den für R⁶ genannten Alkoxy- oder Acyloxygruppen, die sowohl geradkettig als auch verzweigt sein können, sind die Methoxy-, Ethoxy-, Propoxy-Isopropoxy- bzw. die Formyloxy-, Acetyloxy- und Propionyloxy-gruppe besonders bevorzugt.

Von den C₁-C₈-Alkyl- und Alkoxyalkylresten, die für R⁴ stehen können, sind dies vor allem der Methyl-, Ethyl-, Propyl-, Isopropyl-, Cyclopentyl- und Cyclohexylrest bzw. die Alkoxymethyl- bzw. 1- oder 2-Alkoxyethylgruppen mit den genannten Alkylresten; von den C₁-C₈-Acylresten für R⁴ ist insbesondere an den Acetyl-, Propionyl- und Isobutyrylrest gedacht.

Steht R⁴ für die 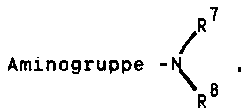 bedeuten R⁷ und R⁸ vorzugsweise je weils einen Methylrest, doch auch dem Ethylrest kommt besondere Bedeutung zu, wobei dann entweder beide Reste am Stickstoffatom für einen Ethylrest oder einer für einen Methyl- und einer für einen Ethylrest stehen. Für den Substituenten R⁹ sind die Methyl-, Ethyl- und 2-(Dimethylamino)ethylgruppe besonders hervorzuheben.

Von den gemäß Formel Iα möglichen Heteroarylresten sind der 3-Thienyl-, 3-Furyl- und 3-Pyrrolylrest bevorzugt mit R¹⁰ in der Bedeutung einer Cyano-Methoxy- oder Dimethylaminogruppe.

Als Heteroarylreste der Formel Iβ kommen erfindungsgemäß insbesondere der 3-oder 4-Pyridyl-, der 5-Pyrimidinyl-, 4-Pyridazinyl- oder Pyrazinylrest infrage. Der Phenylrest der Formel Iγ weist als Substituenten R¹⁰ insbesondere die Cyano-, Methoxy- oder Dimethylaminogruppe auf, wobei sich wiederum diese Substituenten bevorzugt in der p-Position des Phenylringes befinden.

Die folgenden Verbindungen sind erfindungsgemäß insbesondere bevorzugt:
17α-Hydroxy-17β-(3-hydroxypropyl)-11β-[4-(3-pyridinyl)phenyl]-14β-estr-4-en-3-on
11-β-(4-acetylphenyl)-17α-hydroxy-17β-(3-hydroxypropyl)-14β-estr-4-en-3-on
11β-[4-(3-Furanyl)phenyl]-17α-hydroxy-17β-(3-hydroxypropyl]-14β-estr-4-en-3-on
4'-[17α-Hydroxy-17β-(3-hydroxypropyl)-3-oxo-14β-estr-4-en-11β-yl][1,1'-biphenyl]-4-carbonitril
(Z)-11β-(4-Acetylphenyl)-17α-hydroxy-17β-(3-hydroxy-1-propenyl)-14β-estr-4-en-3-on
(Z)-4'-[17α-Hydroxy-17β-(3-hydroxy-1-propenyl)-3-oxo-14β-estr-4-en-11β-yl][1,1'-biphenyl]-4-carbonitril
11β-(4-Acetylphenyl)-17α-hydroxy-17β-(methoxymethyl)-14β-estr-4-en-3-on
11β-(4-Acetylphenyl)-17α-hydroxy-3-oxo-14β-estr-4-en-17β-acetonitril
11β-[4-(3-Furanyl)phenyl]-17α-hydroxy-17β-(3-hydroxypropyl)-14β-estra-4,15-dien-3-on
4'-[17α-Hydroxy-17β-(3-hydroxypropyl)-3-oxo-14β-estra-4,15-dien-11β-yl][1,1'-biphenyl]-4-carbonitril
11β-(4-Acetylphenyl)-17α-hydroxy-17β-methyl-14β-estra-4,15-dien-3-on
17β-Hydroxy-11β-(4-methoxyphenyl)-17α-methylestra-5,14-dien-3-on
11β-[4-(3-Furanyl)phenyl]-17β-hydroxy-17α-methylestra-5,14-dien-3-on
11β-(4-Acetylphenyl)-17β-hydroxy-17α-methylestra-5,14-dien-3-on
11β-(4-Acetylphenyl)-17β-hydroxy-17α-(3-hydroxypropyl)estra-5,14-dien-3-on
4'-[17β-Hydroxy-17α-(3-hydroxypropyl)-3-oxoestra-4,14-dien-11β-yl][1,1'-biphenyl]-4-carbonitril
11β-(4-Acetylphenyl)-17β-hydroxy-17α-(1-propinyl)estra-4,14-dien-3-on
11β-(4-Acetylphenyl)-4',5'-dihydrospiro[estra-4,14-dien-17β,2'(3'H)-furan]-3-on
11β-[4-(3-Furanyl)phenyl]-17β-hydroxy-17α-(3-hydroxypropyl)estra-4,15-dien-3-on
4'-[17β-Hydroxy-17α-(3-hydroxypropyl)-3-oxoestra-4,15-dien-11β-yl][1,1'-biphenyl]4-carbonitril
11β-[4-(3-Furanyl)phenyl]-17β-hydroxy-17α-methylestra-4,15-dien-3-on
11β-(4-Acetylphenyl)-17β-hydroxy-3-oxoestra-4,15-dien-17α-acetonitril
17-Hydroxy-17α-methyl-11β-[4-(3-thienyl)phenyl]estra-4,14-dien-3-on
17β-Hydroxy-11β-(4-methoxyphenyl)-17α-methylestra-4,15-dien-3-on
11β-(4-Acetylphenyl)-17β-hydroxy-17α-methylestra-4,15-dien-3-on
17β-Hydroxy-17α-methyl-11β-[4-(3 pyridinyl)phenyl]estra-4,15-dien-3-on
4'-[17β-Hydroxy-17α-methyl-3-oxoestra-4,15-dien-11β-yl][1,1'-biphenyl]-4-carbonitril
4'-[17β-Methoxy-17α-methyl-3-oxoestra-4,15-dien-11β-yl][1,1'-biphenyl]-4-carbonitril
17β-Hydroxy-11β-(4-methoxyphenyl)-17α-(1-propinyl)estra-4,15-dien-3-on
4'-[17β-Hydroxy-17α-(1-propinyl)-3-oxoestra-4,15-dien-11β-yl][1,1'-biphenyl]-4-carbonitril
11β-[4-(3-Furanyl)phenyl]-17β-hydroxy-17α-(1-propinyl)estra-4,15-dien-3-on
11β-(4-Acetylphenyl)-17β-hydroxy-17α-(1-propinyl)estra-4,15-dien-3-on
4'[4',5'-Dihydro-3-axospiro[estra-4,15-dien-17β,2'(3'H)-furan]-11β-yl][1,1'-biphenyl]-4-carbonitril
(Z)-17β-Hydroxy-17α-3-hydroxy-1-propenyl)-11β-(4-methoxyphenyl)-estra-4,15-dien-3-on
4'-(4',5'-Dihydro-3-oxospiro[estra-4,15-dien-17β,2'(3'H)-furan]-11β-yl][1,1'-biphenyl]-4-carbonitril
11β-(4-Acetylphenyl)-17α-ethinyl-17β-hydroxyestra-4,15-dien-3-on
4'-[17α-Ethinyl-17β-hydroxy-3-oxoestra-4,15-dien-11β-yl][1,1'-biphenyl]-4-carbonitril
11β-[4-(Dimethylamino)phenyl]-17β-hydroxy-17α-methylestra-4,15-dien-3-on

Die Darstellung der erfindungsgemäßen Verbindungen der allgemeinen Formel I ergibt sich aus dem nachstehenden Reaktionsschema:

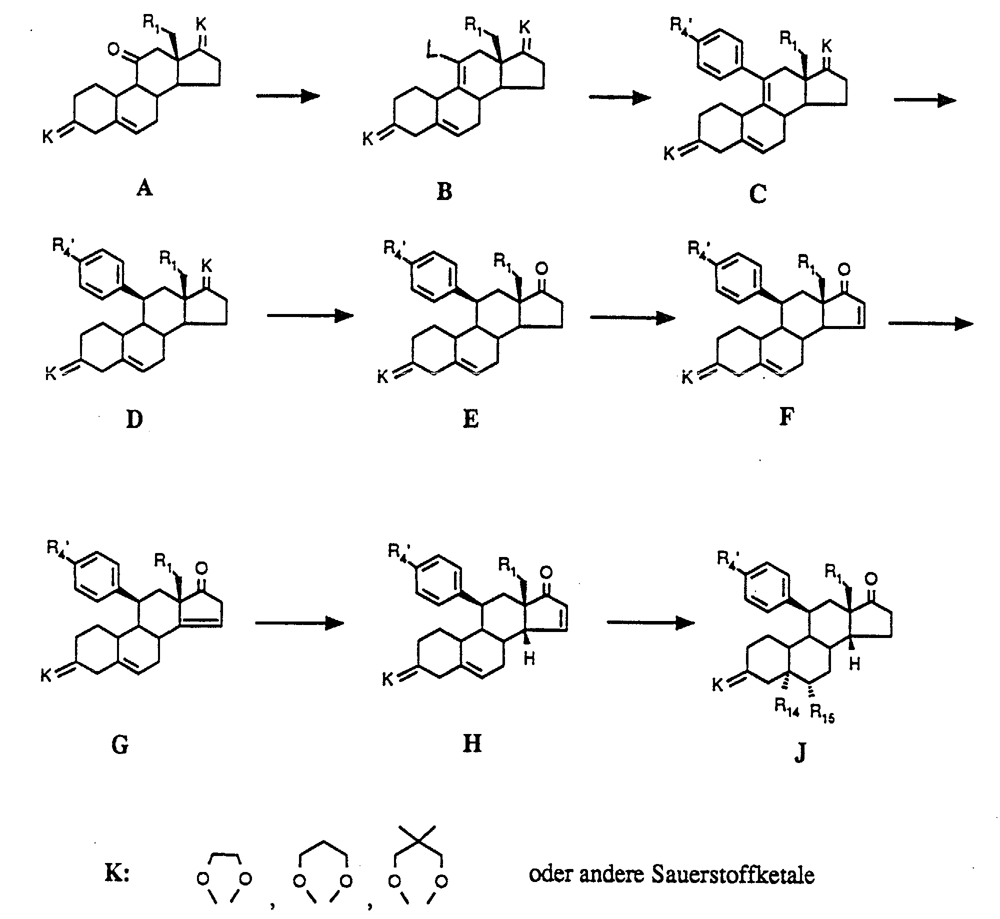

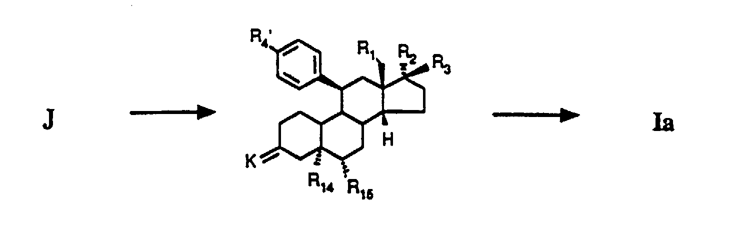

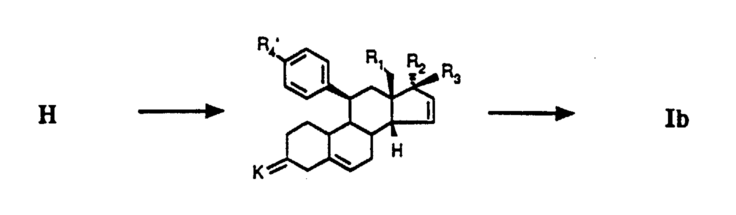

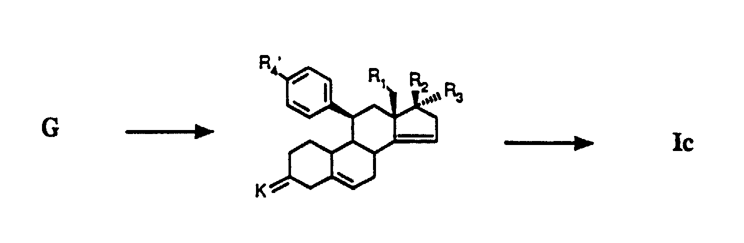

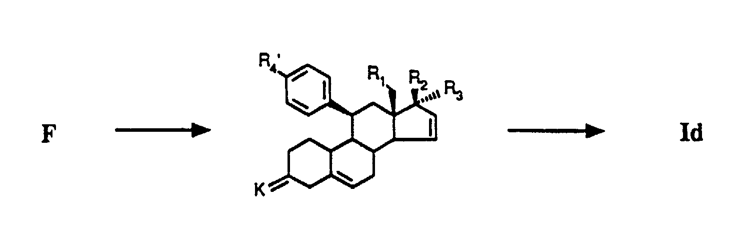

Gemäß der vorliegenden Erfindung wird Verbindung A (Recl. Trav. Chim. BaysPas 107, 331, (1988)) zunächst in eine Verbindung der Formel B überführt, wobei L für eine Perfluoralkylsulfonyloxygruppe CₙF₂ₙ₊₁SO₂O- (n= 1, 2, 3, 4) steht.

Verbindung B wird in Gegenwart einer katalytischen Menge eines Übergangsmetallkatalysators mit einer Arylverbindung der allgemeinen Formel Z 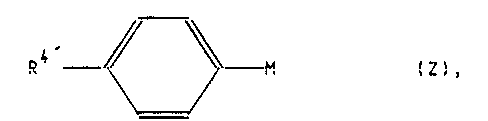 worin M für einen der Reste 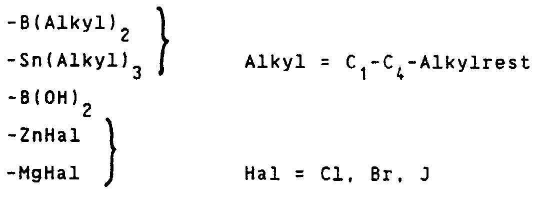 und R^{4'} für einen der unter R⁴ genannten Reste stehen,
zu einer Verbindung der allgemeinen Formel C,
worin R¹ die in Formel I und R^{4'} die in Formel Z angegebene Bedeutung haben und gegebenenfalls, wenn R⁴ in der Formel I eine andere Bedeutung als R^{4'} in der Formel C haben soll, eine Verbindung der allgemeinen Formel C, worin R^{4'} für ein Bromatom steht oder nach Überführung einer für R^{4'} stehenden Methoxygruppe in eine Perfluoralkylsulfonyloxygruppe CₙF₂ₙ₊₁SO₂O- (n=1,2,3,4), mit einer Verbindung der allgemeinen Formel VI

R⁴-M (VI),

worin R⁴ die letztlich für diesen Substituenten in der Formel I gewünschte und M die bereits in Formel Z angegebene Bedeutung haben, umgesetzt.

Für L in der Verbindung B steht vorzugsweise die Trifluormethylsulfonyloxygruppe.
Als Übergangsmetallkatalysator zur Kupplung der Arylverbindung der allgemeinen Formel Z mit der die Abgangsgruppe L aufweisenden Verbindung dient gemäß den Beispielen vorliegender Erfindung Palladiumtetrakistriphenylphosphin (siehe nachstehend angegebene Literatur); genausogut könnte aber Nikkeltetrakistriphenylphosphin oder ähnliche Übergangsmetallkatalysatoren verwendet werden.

Die Variante, daß der letztendlich gewünschte Substituent R⁴ über die Funktionalisierung eines Brom- oder Methoxysubstituenten R^{4'} in der Verbindung C eingeführt wird, ist dann zu wählen, wenn die zu kuppelnde Arylverbindung der allgemeinen Formel Z, worin R^{4'} bereits mit R⁴ identisch ist, nicht zugänglich oder zur Kupplung nicht geeignet ist.
Übergangsmetallkatalysierte Arylkupplungsreaktionen von Verbindungen des Typs der allgemeinen Formel Z mit Verbindungen, die eine Abgangsgruppe tragen, sind beispielsweise beschrieben in: mit -Sn(Alkyl)₃-substituierten Aromaten: J.E. McMurry and S. Mohanraj. Tetrahydron Letters, 24, No. 27, S. 2723-2726, 1983; X. Lu und J. Zhu, Communications, S. 726-727, 1987; Q.-Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters 27, No. 33, S. 3931-3934, 1986; A.M. Echavarren und J.K. Stille, J.Am.Chem.Soc. 1987, 109, S. 5478-5486 und J.Am.Chem.Soc. 1988, 110, S. 1557; mit -B(OH)₂ und -B(OAlkyl)₂-substituierten Aromaten: Y. Hoshino, N. Miyaura und A. Suzuki, Bull.Chem.Soc. Jpn. 61, 3008 (1988); H. Matsubasa, K. Seto, T. Tahara and S. Takahashi; Bull.Chem. Soc. Jpn, 62. 3896 (1989); mit -ZnCl-substituierten Aromaten: R. McCague, Tet. Lett., 28, 701 (1987); A. Arcadi, A. Burini, S. Cacchi, M. Delmastro, F. Marinelli, B. Pietroni, Syn. Les., 1, 1990, S. 47.

Die Verbindungen der allgemeinen Formel 0, die als Ausgangsprodukte für die Herstellung der 10β-H-Steroide der allgemeinen Formel I geeignet sind, lassen sich bequem herstellen, indem eine Verbindung der Formel C,
worin R⁴ und R¹ die in den Formeln angegebene Bedeutung haben, ohne Zerstörung des aromatischen Systems und der 5,6-Doppelbindung zu einer Verbindung der allgemeinen Formel D, worin R⁴ und R¹ die bereits angegebene Bedeutung haben, reduziert wird.
Bei der Reduktion von C bildet sich die 11β-Arylverbindung D (stereoselektive Reduktion).
Zur Reduktion der 9(11)-Doppelbindung in C kommen erfindungsgemäß verschiedene Methoden infrage:
erfindungsgemäß bevorzugt ist die Reduktion mit einem elektropositiven Metall in einem eletronensolvatisierenden Lösungsmittel oder in einem einen Lösungsvermittler enthaltenden Lösungsmittel. Als elektronensolvatisierendes Lösungsmittel kommt in erster Linie Ammoniak inbetracht.

Für die Reduktion genügen bereits equimolare Mengen Reduktionsmittel, es kann jedoch auch ein beträchtlicher Überschuß Reduktionsmittel verwendet werden, ohne daß das aromatische System und/oder die 5,6-Doppelbindung angegriffen werden/wird.
Als elektropositive Metalle sind alle für eine Birch-Reduktion geeigneten Metalle verwendbar. Erfindungsgemäß sind Lithium, Kalium, Natrium und Calcium -und von diesen Lithium insbesondere - bevorzugt.

Selektive Spaltung der Ketoschutzgruppe in 17-Position mit einer schwachen Säure (Essigsäure, Oxalsäure) ergibt die Verbindung E.

Die Zwischenverbindungen der Formel F mit einem ungesättigten D-Ring sind z.B. durch modifizierte Saegusa-Oxidation (Tetrahedron 42 (1986) 297; EP-A 0299 913) der entsprechenden Enolverbindung des 17-Ketons zugänglich.

Basisches Behandeln von Verbindungen des Typs F, z.B. mit Kieselgel/Triethylamin ergibt die Verbindungen G mit Δ¹⁴-Doppelbindung (S. Scholz et al., Liebigs Ann. Chem. 1989, 151). Die Verbindungen lassen sich z.B. über den entsprechenden Trimethylsilylenolether durch Behandeln mit Fluorwasserstoff-Pyridin-Komplex in die entsprechende 14β-H-Verbindung der Formel H überführen.

Verbindungen des Typs H können entweder durch Birch-Reduktion oder mit komplexen Hydriden bzw. Trialkylzinnhydriden unter Kupfer-Katalyse zu Verbindungen der allgemeinen Formel J reduziert werden, wobei in diesem Fall R¹⁴ und R¹⁵ gemeinsam eine Doppelbindung bilden.

Alternativ hierzu können die Verbindungen H auch epoxidiert werden, z.B. mit organischen Persäuren oder Wasserstoffperoxid in Gegenwart von z.B. Hexachloraceton oder Nitrotrifluoracetophenon. Anschließende Reduktion ergibt die Verbindungen J mit R¹⁴ = OH, R¹⁵ = H. Die zuletzt genannte Variante hat den Vorteil, daß Hydrierungen zu gesättigten 17-C-Seitenketten ohne Selektivitätsprobleme durchgeführt werden können.

Verbindungen des Typs H, J, G und F können in Verbindungen der allgemeinen Formeln Ia-d überführt werden.
Hierzu werden zunächst die am 17-C-Atom gewünschten Substituenten R² und R³ eingeführt. Diese Einführung erfolgt analog literaturbekannten Verfahren (z.B. J. Fried, J.A. Edwards, "Organic Reactions in Steroid Chemistry", Van Nostrand Reinhold Company, 1972, Vol. 1 und 2; "Terpenoids and Steroids", Specialist Periodical Report, The Chemical Society, London, Vol. 1-2) durch nucleophile Addition an das C-17-Keton.

Im Falle eines leicht enolisierbaren 17-Ketons, wie z.B. im Fall der Verbindung G, werden Nucleophile unter Zusatz von Cersalzen eingeführt.

Der stereochemische Verlauf der Addition eines Nucleophils an die 17-Ketogruppe der Verbindungen J, H, G und F hängt von der Stellung des Wasserstoffatoms am 14-C-Atom ab. Bei Vorhandensein eines 14β-ständigen H-Atoms in J oder H tritt das Nucleophil in der β-Position ein; befindet sich zwischen C-14 und C-15 eine Doppelbindung (G) oder an C-14 ein α-ständiges H-Atom (F), findet der Eintritt in α-Position statt.

Die Einführung des Substituenten -C≡C-Y als R³, wobei Y die oben angegebene Bedeutung hat, erfolgt mit Hilfe einer metallierten Verbindung der allgemeinen Formel MC≡C-Y', in der Y' eine Alkin- Schutzgruppe, wie zum Beispiel Trimethylsilyl oder tert.-Butyldimethylsilyl, ist.

Die metallorganische Verbindung kann auch in situ gebildet und mit dem 17Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran2'-yloxy)-prop-1-in-1-id, zum 17-[3-(Tetrahydropyran-2'-yl-oxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.
Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19).Als desaktivierte Edelmetallkatalysatoren kommen beispielsweise 10% Palladium auf Bariumsulfat in Gegenwart eines Amins oder 5 % Palladium auf Calciumcarbonat unter Zusatz von Blei(II)acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1Lithium-3-(tetrahydropyran-2'-yloxy)-prop-1(Z)-en (Synthesis 1981, 999) Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogen-propanolen - wobei die Hydroxygruppe im Metallierungsschritt als Alkoholat (Tetrahedron Letters 1978, 3013) oder als geschützte Funktion (J. Org. Chem. 37*,* 1947) vorliegt - zu der 17-(3-Hydroxypropyl)-17β-hydroxy-verbindung bzw. zu der an der terminalen Hydroxygruppe geschützten Verbindung erfolgen. Als Schutzgruppen kommmen zum Beispiel die Ethoxyethyl-, Tetrahydropyranyl- und MethoxymethylGruppen in Frage. Alternativ hierzu kann auch eine 2-Propenylgruppe addiert werden, die dann durch Hydroborierung, vorzugsweise mit sterisch gehinderten Boranen, wie z.B. 9-Borabicyclononan (9-BBN), in die 3-Hydroxypropangruppe überührt wird.
Werden Endprodukte der Formel I gewünscht mit R²/R³ in der Bedeutung von 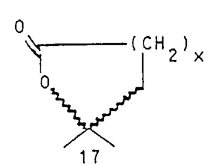 x = 1 oder 2
so wird die 17-(3-Hydroxypropyl)- bzw. 17-(4-Hydroxybutyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968] 900).

Die Darstellung von Endprodukten der Formel I mit R²/R³ in der Bedeutung von 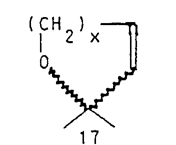 x = 1 oder 2
erfolgt durch Ringschlußreaktion des entsprechenden 17-(3-Hydroxyprop-1-(Z)enyl- bzw. 17-(4-Hydroxybut-1-(Z)-enyl-17-β-hydroxy-Eduktes. Hydrierung des ungesättigten 5- oder 6-Ring-Spiroethers am Palladium/Aktivkohle-Kontakt führt zu den gesättigten Spiroethern.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.
Freie Hydroxygruppen können in an sich bekannter Weise alkyliert oder acyliert werden.

Der nächste Reaktionsschritt dient dem Aufbau des Substituenten R⁴ bzw. R^{4'} in p-Stellung am 11β-Phenylring.
Diese Vorgehensweise ist dann erforderlich, wenn R⁴ nicht direkt bei der Kupplung der Verbindung B mit der Arylverbindung Z zur Verbindung C eingeführt wird.

Als Ausgangspunkt für diesen Aufbau dient die Verbindung J, H, G oder F, worin R⁴ = OH ist, die aus der entsprechenden Methoxyverbindung durch Etherspaltung, beispielsweise mit Natriumethanthiolat in einem Lösungsmittel wie Dimethylformamid, erhältlich ist.

Durch Umsetzung der Hydroxyverbindung mit einem Perfluor-(C₁-C₄)-alkylsulfonsäureanhydrid oder -halogenid in Gegenwart einer Base wie Pyridin oder 4-(Dimethylamino)-pyridin gelangt man zur entsprechenden 11β-[4-(perfluoralkylsulfonyloxy)phenyl]-Verbindung (P.J. Stang, M. Hanack und L.R. Subramanian, Synthesis 85, (1982)).
Bei der sich anschließenden Kupplung der 11β-Arylverbindung mit R^{4"}-Sn(Al-kyl)₃ oder R^{4"}-BL₂ wird entweder so vorgegangen, daß in einer übergangsmetallkatalysierten Reaktion (vorzugsweise Pd°) die Perfluoralkylsulfonatabgangsgruppe unter im wesentlichen fast gleichzeitiger Substitution durch den gewünschten Substituenten oder dessen Vorstufe verdrängt wird (Arylkupplungen mit Zinnverbindungen: J.E. McMurry and S. Mohanraj, Tetrahedron Letters, 24, No. 27, S. 2723-2726, 1983; X. Lu und J. Zhu, Communications, S. 726-727, 1987; Q. -Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters, 27, No. 33, S. 3931-3934, 1986; A.M. Echavarren und J.K. Stille, J. Am. Chem. Soc. 1987, 109, S. 5478-5486; mit Borverbindungen: Synthesis 936 (1984), Chem.Pharm.Bull. 33, 4755-4763 (1985); J.org.Chem.49, 5237-5243 (1984); Bull.Chem.Soc.Jpn.61, 3008-3010β (1988) oder es wird aus der Perfluoralkylsulfonat-Verbindung intermediär und übergangsmetallkatalysiert eine entsprechende Tri-organylstannyl-, vorzugsweise Tri-n-alkylstannyl-Verbindung hergestellt [J.K. Stille, Angew. Chem. 98 (1986), S. 504-519]. Diese wird anschließend in einer Eintopf-Reaktion mit einem halogen-, vorzugsweise brom-oder jodsubstituierten carbocyclischen oder heterocyclischen Aromaten [Y. Yamamoto, Y. Azuma, H. Mitoh, Communications, S. 564-565, 1986; T. J. Bailey, Tetrahedron Letters, 27, No. 37, S. 4407-4410, 1986], der gegebenenfalls noch weitere Substituenten tragen kann, umgesetzt; der 11β-Phenylrest weist darin dann die gewünschte bzw. einen Vorläufer der gewünschten Substitution auf.
Zahlreiche derartige Umsetzungen mit Steroiden, in denen eine Trifluormethansulfonat-Gruppe sich in 4-Stellung eines 11β-Phenylringes befindet, sind in der EP-A-0283428 beschrieben.
Freie Hydroxygruppen können in an sich bekannter Weise alkyliert oder acyliert werden.
Dialkylamine können durch geeignete Oxidationsmittel (z.B. Wasserstoffperoxid bzw. Persäuren) in die gewünschten N-Oxide [siehe z.B. Kontakte (Darmstadt) 1986,3,S.12] überführt werden.

Verbindungen mit einem Dialkylamin-Substituenten am 11β-Phenylring können durch Reaktion mit Bromcyan in aprotischen Lösungsmitteln wie zum Beispiel Dioxan, Benzol oder Toluol bei erhöhter Temperatur (Amin-Abbau nach Braun) analog den
zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)derivate überführt werden.

Diese werden je nach der letztlich gewünschten Bedeutung von 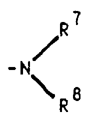 im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit z.B. Lithiumaluminiumhydrid zum neuen Dialkylaminderivat reduziert (s. DE 36 23 038). Gegebenenfalls kann auch zuerst der Substituent R⁴ aufgebaut werden und anschließend die Einführung der Substituenten R² und R³ vorgenommen werden, je nachdem, ob die Verfahrensbedingungen des zweiten Reaktionsschrittes die zuerst eingeführten oder aufgebauten Substituenten beeinträchtigen.

Noch vorhandene Schutzgruppen werden nach gängigen Methoden abgespalten.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40°C in die Oxime (Formel I mit X in der Bedeutung der Hydroxyiminogruppierung 〉N~OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]non-5en (DBN) und 1,8-Diazabicyclo[5.4.0]undec-7-en (DBU), wobei Pyridin bevorzugt ist.

Die Entfernung der 3-Oxogruppe zu einem Endprodukt der allgemeinen Formel I mit X in der Bedeutung von 2 Wasserstoffatomen kann z.B. nach der in der DE-A-2805490 angegebenen Vorschrift durch reduktive Spaltung des Thioketals erfolgen.

Die neuen Verbindungen der allgemeinen Formel I sowie deren pharmakologisch verträgliche Additionssalze mit Säuren sind wertvolle Pharmaka. So verfügen sie über eine starke Affinität zum Gestagenrezeptor und besitzen überraschend starke antigestagene sowie antiglucocorticoide, antimineralcorticoide und antiandrogene Eigenschaften. Diese wichtigen biologischen Wirksamkeiten können für medizinische Zwecke genutzt werden.

Die starke Affinität zum Gestagenrezeptor ergibt sich aus dem bekannten, u.a. in der EP-A-0 190 759 beschriebenen Gestagenrezeptor-Bindungstest. Folgende Verbindungen wurden untersucht:
17β-Hydroxy-11β-(4-methoxyphenyl)-17α-methylestra-4,14-dien-3-on (A)
11β-[4-(3-Furanyl)Phenyl]-17β-hydroxy-17α-methylestra-4,14-dien-3-on (B)
11β-(4-Acetylphenyl)-17β-hydroxy-17α-methylestra-4,14-dien-3-on (C)
17β-Hydroxy-17α-methyl-11β-[4-(3-thienyl)phenyl]estra-4,14-dien-3-on (D)
11β-[4-Acetylphenyl)-17β-hydroxy-17α-methylestra-4,15-dien-3-on (E)
11β-[(3-Furanyl)phenyl]-17β-hydroxy-17α-methylestra-4,15-dien-3-on (F)
17β-Hydroxy-11β-(4-methoxyphenyl)-17α-methylestra-4,15-dien-3-on (G)
17β-Hydroxy-17α-methyl-11β-[4-(3-pyridinyl)phenyl]estra-4,15-dien-3-on (H)
4'-[17β-Hydroxy-17α-methyl-3-oxoestra-4,15-dien-11β-yl]-[1,1'-biphenyl]-4-carbonitril (I)

Die untersuchten Verbindungen weisen folgende Kompetitionsfaktoren auf (Bezugsubstanz: ³H-Progesteron; Gewebe aus Kaninchenuterus).

| | | | | | | | | | |
|---|---|---|---|---|---|---|---|---|---|
| Testverbindung | A | B | C | D | E | F | G | H | I |
| Kompetitionsfaktor K | 2,5 | 2,6 | 4,0 | 3 | 2,6 | 1 | 3,2 | 0,6 | 0,7 |

Zur Kennzeichnung der antigestagenen Wirkung wurde die abortive Wirkung an graviden Ratten nach dem in der EP-A-0 283 428 beschriebenen Test bestimmt.

Untersucht wurden die Verbindungen B, C, E und F (siehe Tabelle 1). Applikation der Testverbindungen an d5-d7 p.c. p.o.; Autopsie an d9 p.c.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.
Die neuen Verbindungen können außerdem zur Behandlung der Endometriose dienen.
Sie können auch gegen hormonelle Unregelmäßigkeiten, zur Menstruationsauslösung und zur Geburtseinleitung eingesetzt werden. Außerdem können sie für die Behandlung von hormonabhängigen Carcinomen eingesetzt werden.

Die erfindungsgemäßen Verbindungen der allgemeinen Formel I sowie deren pharmakologisch verträgliche Additionssalze mit Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren pharmakologisch verträgliche Additionssalze mit Säuren mit antiandrogener Aktivität können bei der Behandlung der Hypertrophie und des Prostatakarzinoms verwendet werden. Sie ermöglichen weiterhin eine spezifische Therapie von Androgenisierungserscheinungen bei Frauen: die pathologische Behaarung bei Hirsutismus, die androgenetische Alopezie sowie die gesteigerte Talgdrüsenfunktion bei Akne und Seborrhoe sind günstig beeinflußbar.

Die Erfindung betrifft somit auch Arzneimittel auf Basis der Verbindungen der allgemeinen Formel I sowie deren pharmakologisch verträglichen Additionssalze mit Säuren, gegebenenfalls zusammen mit den üblichen Hilfs-und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.
Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens^{(R)} oder Myrj^{(R)}, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.
Die Erfindung betrifft somit auch pharmazeutische Zusammensetzungen, die als Wirkstoff zumindest eine erfindungsgemäße Verbindung oder eines ihrer pharmakologisch verträglichen Additionssalze mit Säuren enthalten. Als Additionssalze der erfindungsgemäßen Produkte mit Säuren sind insbesondere die Hydrochloride und die Methansulfonate zu nennen. Eine Dosiseinheit enthält etwa 1-100 mg Wirkstoff(e).
Die Dosierung der erfindungsgemäßen Verbindungen liegt beim Menschen bei etwa 1-1000 mg pro Tag.

Die nachfolgenden Ausführungsbeispiele dienen der näheren Erläuterungen der Erfindung:

### Beispiel 1

### 11β-(4-Acetylphenyl)-17α-hydroxy-17β-(3-hydroxypropyl)-14β-estr-4-en-3-on

### a) 3,3;17,17-Bis[1,2-ethandiylbis(oxy)]-11-[[(trifluormethyl)sulfonyl]oxy]estra-5,9(11)-dien

26,1 g 3,3;17,17-Bis[1,2-ethandiylbis(oxy)]estr-5-en-11-on werden in 350 ml absolutem Methylenchlorid gelöst und unter Schutzgas mit 18 ml 2,6-Di-tertiär-butylpyridin versetzt. Nach Kühlen dieser Lösung auf 0°C werden 12,9 ml Tkifluormethansulfonsäureanhydrid langsam zugetropft. Danach wird das Reaktionsgemisch 20 Stunden bei Raumtemperatur nachgerührt. Zur Aufarbeitung wird es auf gesättigte Natriumhydrogencarbonatlösung gegossen, die organische Phase abgetrennt und die wässrige mit Methylenchlorid nachextrahiert. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Ethylacetat/Hexan werden neben 16,4 ml 2,6-Di-tertiär-butylpyridin und 5,1 g 3,3;17,17-Bis[1,2-ethandiylbis(oxy)]estr-5-en-11-on 27 g 3,3;17,17-Bis[1,2-ethandiylbis(oxy)]-11-[[(trifluormethyl)sulfonyl]oxy]estra-5,9(11)-dien als weißer Schaum erhalten.

[α]²⁰_{D} = + 104 ° (CHCl₃;c=0.505)

¹H-NMR(CDCl₃) δ: 5,58 dbr (J=5 Hz,1H,H-6); 3,7-4,0 m (8H,m,Ketale); 2,88 dbr (J=11 Hz,1H,H-10); 2,74 dtr (J=16, 2.5 Hz,1H,H-12); 2,18-2,33 m (2H,H-4); 0,84 s (3H,H-18).

### b) 3,3;17,17-Bis[1,2-ethandiylbis(oxy)]-11-(4-methoxyphenyl)-estra-5,9(11)-dien

27 g 3,3;17,17-Bis[1,2-ethandiylbis(oxy)]-11-[[(trifluormethylsulfonyl]oxy]estra-5,9(11)dien werden in einem Gemisch aus 450 ml absolutem Toluol und 210 ml absolutem Ethanol gelöst und nacheinander mit 3,1 g Palladiumtetrakistriphenylphosphin, 4,5 g Lithiumchlorid, 70 ml 2 molarer Natriumcarbonatlösung und 9 g 4-Methoxyphenylboronsäure versetzt. Das Reaktionsgemisch wird dann 2 Stunden bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrokknet und im Vakuum eingeengt. Der Rückstand wird an Kieselgel mit einem Gemisch aus Ethylacetat/Hexan chromatographiert. Man erhält 24 g 3,3;17,17-Bis[1,2-ethandiylbis(oxy)]-11-(4-methoxyphenyl)-estra-5,9(11)-dien als weißen Schaum.

### c) 3,3; 17,17-Bis[1,2-ethandiylbis(oxy)]-11β-(4-methoxyphenyl)estr-5-en

1000 ml Ammoniak werden bei -70 °C kondensiert und mit 1,80 g Lithium versetzt. Nach Auftreten der charakteristischen Blaufärbung werden 24 g 3,3;17,17-Bis[1,2-ethandiylbis(oxy)]-11β-(4-methoxyphenyl)-estra-5,9(11)-dien gelöst in 500 ml absolutem Tetrahydrofuran zugetropft. Nach 20-minütigem Nachrühren zersetzt man das überschüssige Lithium durch Zugabe von Wasser, dampft das Ammoniak ab, gießt das Reaktionsgemisch auf gesättigte Ammoniumchloridlösung und extrahiert die wässrige Phase mit Ethylacetat Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden 19,6 g 3,3;17,17-Bis[1,2-ethandiylbis(oxy)]-11β-(4-methoxyphenyl)estr-5-en und 1,8 g 3,3;17,17-Bis[1,2-ethandiylbis(oxy)]-11-(4-hydroxyphenyl)estra-5,9(11)-dien als weiße Schäume isoliert.

### d) 3,3-[1,2-Ethandiylbis(oxy)]-11β-(4-methoxyphenyl)estr-5-en-17-on

60 g Kieselgel werden in 130 ml Methylenchlorid suspendiert mit 5,9 ml gesättigter Oxalsäurelösung versetzt und 15 Minuten nachgerührt. Zu dieser Suspension werden 19,6 g 3,3;17,17-Bis[1,2-ethandiylbis(oxy)]-11β-(4-methoxyphenyl)estr-5-en gegeben und das Reaktionsgemisch bei Raumtemperatur 4 Stunden nachgerührt. Anschließend wird es über eine Fritte abgesaugt, der Frittenrückstand mit Methanol/Methylenchlorid nachgewaschen und das so erhaltene Filtrat mit gesättigter Natriumhydrogencarbonatlösung ausgeschüttelt. Die organische Phase wird über Natriumsulfat getrocknet und am Vakuum eingeengt. Der Rückstand wird an Kieselgel mit einem Gemisch aus. Ethylacetat/Hexan chromatographiert. Man erhält 13,77 g 3,3-[1,2-Ethandiylbis(oxy)]-11β-(4-methoxyphenyl)estr-5en-17-on als weißen Schaum.

### e) 3,3-[1,2-Ethandiylbis(oxy)]-11β-(4-methoxyphenyl)-17-[(trimethylsilyl)oxy]estra-5,16-dien

Aus 14,07 ml Diisopropylamin und 72 ml einer 1,6 molaren Lösung von n-Butyllithium in Hexan wird bei -30°C Lithiumdiisopropylamid erzeugt. 3,3-[1,2-Ethandiylbis(oxy)]11β-(4-methoxyphenyl)estr-5-en-17-on (28,28 g gelöst in 250 ml absolutem Tetrahydrofuran) wird hinzugetropft. Anschließend läßt man 15 min bei 0°C nachrühren. Dann werden 24,3 ml Trimethylchlorsilan hinzugetropft. Man rührt weitere 15 min bei 0°C, gießt die Reaktionslösung auf gesättigte Natriumhydrogencarbonatlösung und extrahiert mit Ethylacetat. Die organische Phase wird mit gesättigter Ammoniumchloridlösung sowie mit gesättigter Natriumchloridlösung gewaschen, über Natriumsulfat getrocknet und im Vakuum eingeengt. Das Rohprodukt wird aus Acetonitril umkristallisiert. Man erhält 25,65 g 3,3-[1,2-Ethandiylbis(oxy)-11β-(4-methoxyphenyl)-17-[(trimethylsilyl)oxy]estra-5,16-dien.

### f) 3,3-[1,2-Ethandiylbis(oxy)]-11β-(4-methoxyphenyl)estra-5,15-dien-17-on

25,65 g 3,3-[1,2-Ethandiylbis(oxy)]-11β-(4-methoxyphenyl)-17-[(trimethylsilyl)oxy]estra-5,16-dien werden in 390 ml Acetonitril gelöst Unter Argon versetzt man mit 17,36 g Palladium@)acetat und läßt über Nacht bei Raumtemperatur rühren. Anschließend wird die Reaktionslösung über Celite filtriert und eingeengt. Das Rohprodukt wird durch Säulenchromatographie an Kieselgel mit Hexan/Ethylacetat gereinigt. Man erhält 16,6 g 3,3-[1,2-Ethandiylbis(oxy)]-11β-(4-methoxyphenyl)estra-5,15-dien- 17-on.

¹H-NMR (CDCl₃): δ= 7,53 dd (J=6, 1 Hz, 1H, H-15); 7,25 d (J=10 Hz, 2H, Ar); 6,80 d (J=10 Hz, 2H, Ar); 5,98 dd (J=6, 3 Hz, 1H, H-16); 5,57 dbr (J=5 Hz, 1H, H-6); 3,87-4,00 (4H, Ketal); 3,79 s (3H, OMe); 3,51 ddbr (J=5, 6 Hz, 1H, H-11); 0,90 s (3H, H-18)

### g) 3,3-[1,2-Ethandiylbis(oxy)]-11β-(4-methoxyphenyl)estra-5,14-dien-17-on

16,6 g (39,47 mmol) 3,3-[1,2-Ethandiylbis(oxy)]-11β-(4-methoxyphenyl)estra-5,15-dien-17-on werden in 3 l Ethylacetat/Hexan (9:1) gelöst. Unter Argon werden 1,3 kg Kieselgel und 240 ml Triethylamin hinzugegeben. Der Ansatz wird 60 h bei Raumtemperatur nachgerührt, Anschließend wird die Reaktionslösung filtriert und im Vakuum eingeengt. Das Rohprodukt wird durch Säulenchromatographie (Hexan/Ethylacetat + 1% Triethylamin) gereinigt. Man erhält 10,04 g 3,3-[1,2-Ethandiylbis(oxy)]-11β-(4-methoxyphenyl)estra-5,14-dien-17-on als weißen Schaum.

¹H-NMR (CDCl₃): δ= 7,33 d (J=10 Hz, 2H, Ar); 6,83 d (J=10 Hz, 2H, Ar); 5,60 m (2H, H-6, H-15); 3,90-4,01 m (4H, Ketal); 3,80 s (3H, OMe); 3,39 ddbr (J=7,5 Hz, 1H, H-11); 0,80 s (3H, H-18)

### h) 3,3-[1,2-Ethandiylbis(oxy)]-11β-(4-methoxyphenyl)-14β-estra-5,15-dien-17-on

Aus 5,03 ml Diisopropylamin und 25,34 ml n-Butyllithium (1,6 molare Lösung in Hexan) wird bei -30°C Lithiumdiisopropylamid dargestellt. Eine Lösung von 10 g 3,3-[1,2-Ethandiylbis(oxy)]-11β-[4-methoxyphenyl)estra-5,14-dien-17-on in 200 ml absolutem Tetrahydrofuran wird hinzugetropft. Man läßt 15 min bei 0°C rühren und tropft dann 8,61 ml Trimethylchlorsilan hinzu. Es wird weitere 15 min bei 0°C nachgerührt, Anschließend kühlt man die Reaktionslösung auf -78°C und tropft 6 ml Fluorwasserstoff-Pyridin-Komplex hinzu. Dann wird 2 Stunden bei -40°C nachgerührt. Anschließend gießt man die Reaktionslösung auf gesättigte Natriumhydrogencarbonatlösung, extrahiert mit Ethylacetat und wäscht die organische Phase mit gesättigter Natriumchloridlösung Es wird über Natriumsulfat getrokknet und im Vakuum eingeengt. Das Rohprodukt wird aus Diisopropylether umkristallisiert. Man erhält 7,4 g 3,3-[1,2-Ethandiylbis(oxy)]-11β-(4-methoxyphenyl)-14β-estra-5,15-dien-17-on als weiße Kristalle.

¹H-NMR (CDCl₃): δ= 7,78 dd (J=6, 2 Hz, 1H, H-15); 7,02 d (J=9 Hz, 2H, Ar); 6,77 d (J=9 Hz, 2H, Ar); 6,36 dd (J=6, 1.5 Hz, 1H, H-16); 5,50 m (1H, H-6); 3,78-3,95 m (4H, Ketal); 3,76 s (3H, OMe); 2,73 ddd (J=11.5, 10.5, 6 Hz, 1H, H-11); 2,62 m (1H, H-14); 1,10 s (3H, H-18)

### i) 3,3-[1,2-Ethandiylbis(oxy)]-11β-(4-methoxyphenyl)-5,6α-epoxy-5α,14β-estr-15-en-17-on

7,40 g 3,3-[1,2-Ethandiylbis(oxy)]-11β-(4-methoxyphenyl)-14β-estra-5,15-dien-17-on werden in 200 ml absolutem Methylenchlorid gelöst. Man kühlt auf 0°C und versetzt mit 5,3 ml gesättigter Natriumhydrogencarbonatlösung, sowie 1,69 g m-Nitrotrifluoracetophenon. Anschließend werden langsam 7 ml einer 30%igen H₂O₂-Lsg hinzugetropft. Man rührt 7 Tage bei Raumtemperatur. Danach wird die Reaktionslösung vorsichtig bei leichter Kühlung mit gesättigter Natriumthiosulfatlösung versetzt. Man läßt weitere 30 min bei Raumtemperatur rühren und extrahiert mit Methylenchlorid. Die organische Phase wird zweimal mit 5%iger Natriumhydroxidlösung, sowie einmal mit gesättigter Natriumchloridlösung gewaschen und über Natriumsulfat getrocknet. Das Rohprodukt wird über eine Säulenchromatographie an Kieselgel mit einem Gemisch aus Hexan und Ethylacetat gereinigt. Man erhält 2,55 g Ausgangsmaterial und 4,16 g 3,3-[1,2-Ethandiylbis(oxy)]-11β-(4-methoxyphenyl)-5,6α-epoxy-5α,14β-estr-15-en-17-on.

¹H-NMR (CDCl₃): δ= 7,72 dd (J=6, 2 Hz, 1H, H-15); 7,06 d (J=9 Hz, 2H, Ar); 6,78 d (J=9 Hz, 2H, Ar); 6,38 dd (J=6, 1.5 Hz, 1H, H-16); 3,82-3,97 m (4H, Ketal); 3,78 s (3H, OMe); 2,94 dbr (J=4.5, 1H, H-6); 2,76 ddd (J=12.5, 9.5, 6 Hz, 1H, H-11); 2,68 m (1H, H-14); 1,04 s (3H, H-18)

### k) 3,3-[1,2-Ethandiylbis(oxy)]-11β-(4-methoxyphenyl)-5α,14β-esuan-5,17α-diol

4,16 g 3,3-[1,2-Ethandiylbis(oxy)]-11β-(4-methoxyphenyl)-5,6α-epoxy-5α,14β-estr-15-en-17-on werden in 200 ml absolutem Ethanol gelöst Man versetzt vorsichtig mit 5,54 g Natriumborhydrid und kocht 1 h unter Rückfluß. Anschließend wird die Reaktionslösung in Wasser gegossen. Es wird mit Methylenchlorid extrahiert. Die organische Phase wird mit gesättigter Natriumchloridlösung gewaschen. Man trocknet über Natriumsulfat und engt im Vakuum ein. Es werden 3,98 g 3,3-[1,2-Ethandiylbis(oxy)]-11β-(4-methoxyphenyl)-5α,14β-estran-5,17α-diol. Das Rohprodukt wird ohne Aufreinigung in die nächste Stufe eingesetzt.

¹H-NMR (CDCl₃): δ= 7,33 d (J=9, 2H, Ar); 6,28 d (J=9, 2H, Ar); 3,85-3,98 m (4H, Ketal); 3,80 s (3H, OMe); 3,58 dd (J=9, 7.5 Hz, 1H, H-17); 3,12 ddbr (J=7, 6 Hz, 1H, H-11); 0,72 s (3H, H-18)

### l) 3,3-[1,2-Ethandiylbis(oxy)]-11β-(4-methoxyphenyl)-5-hydroxy-5α,14β-estran-17-on

Zu einer Mischung aus 35,6 ml Pyridin und 250 ml Methylenchlorid werden bei 0°C 5,50 g Chromtrioxid gegeben. Man rührt 30 min nach und tropft dann eine Lösung von 3,98 g 3,3-[1,2-Ethandiylbis(oxy)]-11β-(4-methoxyphenyl)-5α,14β-estran-5,17α-diol in 70 ml Methylenchlorid hinzu. Anschließend läßt man 1,5 h bei ca. 10°C rühren. Die Reaktionslösung wird zweimal mit 5%iger Natriumhydroxidlösung, sowie einmal mit gesättigter Natriumchloridlösung gewaschen. Man trocknet über Natriumsulfat und engt im Vakuum ein. Das Rohprodukt wird durch Säulenchromatographie an Kieselgel mit einem Gemisch aus Hexan/ Ethylacetat gereinigt. Man erhält 3,53 g 3,3-[1,2-Ethandiylbis(oxy)]-11β-(4-methoxyphenyl)-5-hydroxy-5α,14β-estran-17-on.

¹H-NMR (CDCl₃): δ= 7,31 d (J=10, 2H, Ar); 6,78 d (J=10, 2H, Ar); 3,85-4,00 m (4H, Ketal); 3,80 s (3H, OMe); 3,10 ddbr (J=7, 6 Hz, 1H, H-11); 0,76 s (3H, H-18)

### m) 3,3-[1,2-Ethandiylbis(oxy)]-11β-(4-methoxyphenyl)-17β-[3-[(tetrahydro-2H-pyran-2-yl)oxy]-1-propinyl]-5α,14β-estran-5,17α-diol

Aus 11,23 g 3-[(Tetrahydro-2*H*-pyran-2-yl)oxy]-1-propin in 350 ml absolutem Tetrahydrofuran und 50,7 ml einer 1,6 molaren Lösung von Butyllithium in Hexan stellt man bei 0°C die lithiumorganische Verbindung her. Anschließend wird eine Lösung von 3,53 g 3,3-[1,2-Ethandiylbis(oxy)]-11β-(4-methoxyphenyl)-5-hydroxy-5α,14β-estran-17-on in 70 ml absolutem Tetrahydrofuran hinzugetropft. Man läßt 1 h bei 0°C nachrühren, versetzt mit gesättigter Ammoniumchloridlösung und extrahiert mit Ethylacetat Die organische Phase wird mit gesättigter Natriumchloridlösung gewaschen und über Natriumsulfat getrocknet. Es wird im Vakuum eingeengt. Das Rohprodukt wird durch eine Säulenchromatographie an Kieselgel mit einem Gemisch aus Hexan und Ethylacetat gereinigt. Man erhält 4,54 g 3,3-[1,2-Ethandiylbis(oxy)]-11β-(4-methoxyphenyl)-17β-[3-[(tetrahydro-2*H*-pyran-2-yl)oxy]-1-propinyl]5α,14β-estran-5,17α-diol.

¹H-NMR (CDCl₃): δ= 7,33 d (J=9 Hz, 2H, Ar); 6,79 d (J=9 Hz, 2H, Ar); 4,78 m (1H, THP); 4,28 sbr (2H, CH₂OTHP); 3,85-4,00 m (4H, Ketal); 3,50 m (1H, THP); 3,15 m (1H, THP); 0,83 s (3H, H-18)

### n) 3,3-[1,2-Ethandiylbis(oxy)]-11β-(4-methoxyphenyl)-17β-[3-[(tetrahydro-2H-pyran-2-yl)oxy]propyl]-5α,14β-estran-5,17α-diol

4,54 g 3,3-[1,2-Ethandiylbis(oxy)]-11β-(4-methoxyphenyl)-17β-[3-[(tetrahydro-2*H*-pyran-2-yl)oxy]-1-propinyl]-5α,14β-estran-5,17α-diol werden in 300 ml Tetrahydrofuran/Ethanol (1:1) gelöst. Man versetzt im Argongegenstrom mit 940 mg Palladium auf Calciumcarbonat und setzt die Apparatur unter Wasserstoff. Man läßt 3 Stunden bei Raumtemperatur reagieren. Dann wird die Reaktionslösung über Celite filtriert und eingeengt. Das Rohprodukt wird durch Säulenchromatographie an Kieselgel mit einem Gemisch aus Hexan/Ethylacetat gereinigt Man erhält 4,29 g 3,3-[1,2-Ethandiylbis(oxy)]-11β-(4-methoxyphenyl)-17β-[3-[(tetrahydro-2*H*-pyran-2-yl)oxy]propyl]-5α,14β-estran-5,17α-diol.

¹H-NMR (CDCl₃): δ= 7,31 d (J=9Hz, 2H, Ar); 6,78 d (J=9Hz, 2H, Ar); 4,58 m (1H, THP); 3,70-3,95 (6H); 3,80 s (3H, OMe); 3,35-3,55 m (2H); 3,16 ddbr (J=7, 5 Hz, 1H, H-11); 0,65 s (3H, H-18); 0,63 s (3H, H-18) (isomere THP-Ether)

### o) 3,3-[1,2-Ethandiylbis(oxy)]-11β-(4-hydroxyphenyl)-17β-[3-[(tetrahydro-2H-pyran-2-yl)oxy]propyl]-5α,14β-estran-5,17α-diol

4,29 g 3,3-[1,2-Ethandiylbis(oxy)]-11β-(4-methoxyphenyl)-17β-[3-[(tetrahydro-2*H*-pyran-2-yl)oxy]propyl]-5α,14β-estran-5,17α-diol werden in 45 ml absolutem Dimethylformamid gelöst. Man versetzt mit 2,05 g Natriummethanthiolat und kocht 1,5 Stunden unter Rückfluß. Anschließend wird die Reaktionslösung auf 100 ml eiskalte wäßrige Natriumchloridlösung gegossen und über Nacht nachgerührt. Es wird abgesaugt und das Filtrat mehrmals mit Wasser gewaschen. Man nimmt in Methylenchlorid auf, wäscht mit gesättigter Natriumchloridlösung, trocknet über Natriumsulfat und engt im Vakuum ein. Das Rohprodukt wird durch Säulenchromatographie an Kieselgel mit einem Gemisch aus Hexan/Ethylacetat gereinigt. Man erhält 2,42 g 3,3-[1,2-Ethandiylbis(oxy)]-11β-(4-hydroxyphenyl)-17β-[3-[(tetrahydro-2*H*-pyran-2-yl)oxy]propyl]-5α,14β-estran-5,17α-diol.

¹H-NMR (CDCl₃): δ= 7,25 d (J=9 Hz, 2H, Ar); 6,70 d (J=9 Hz, 2H, Ar); 4,59 m (1H, THP); 3,68-3,97 m (6H); 3,35-3,55 m (2H); 3,13 m (1H, H-11); 0,63 s (3H, H-18); 0,62 s (3H, H-18) (isomere THP-Ether)

### p) 3,3-[1,2-Ethandiylbis(oxy)]-17β-[3-[(tetrahydro-2H-pyran-2-yl)oxy]propyl]-11β-[4-[[(trifluormethyl)sulfonyl]oxy]phenyl]-5α,14β-estran-5,17α-diol

2,42 g 3,3-[1,2-Ethandiylbis(oxy)]-11β-(4-hydroxyphenyl)-17β-[3-[(tetrahydro-2*H*-pyran-2-yl)oxy]propyl]-5α,14β-estran-5,17α-diol werden in 45 ml absolutem Methylenchlorid gelöst. Man versetzt mit 2,59 g 4-Dimethylaminopyridin und kühlt auf -78°C. Anschließend werden 0,92 ml Trifluormethansulfonsäureanhydrid langsam hinzugetropft. Es wird 6 Stunden bei -78°C nachgerührL Dann wird die Reaktionslösung auf gesättigter Natriumhydrogencarbonatlösung gegossen. Es wird mit Methylenchlorid extrahiert. Man wäscht die organische Phase mit gesättigter Natriumchloridlösung, trocknet über Natriumsulfat und engt im Vakuum ein. Das Rohprodukt wird durch Säulenchromatographie an Kieselgel mit einem Gemisch aus Hexan/Ethylacetat gereinigt. Man erhält 2,15 g 3,3-[1,2-Ethandiylbis(oxy)]-17β-[3-[(tetrahydro-2*H*-pyran-2-yl)oxy]propyl]-11β-[4-[[(trifluormethyl)sulfonyl]oxy]phenyl]-5α,14β-estran-5,17α-diol.

¹H-NMR (CDCl₃): δ= 7,49 d (J=9 Hz, 2H, Ar); 7,15 d (J=9 Hz, 2H, Ar); 4,58 m (1H, THP); 3,70-3,98 m (6H); 3,33-3,55 m (2H); 3,24 m (1H, H-11); 0,60 s (3H, H-18); 0,61 s (3H, H-18) (isomere THP-Ether)

### q) 3,3-[1,2-Ethandiylbis(oxy)]-11β-[4-(1-ethoxyethenyl)phenyl]-17β-[3-[(tetrahydro-2H-pyran-2-yl)oxy]propyl]-5α,14β-estran-5,17α-diol

1,08 g 3,3-[1,2-Ethandiylbis(oxy)]-17β-[3-[(tetrahydro-2*H*-pyran-2-yl)oxy]propyl]-11β-[4-[[(trifluormethyl)sulfonyl]oxy]phenyl]-5α,14β-estran-5,17α-diol werden in 15 ml Dioxan gelöst. Man gibt 0,67 ml (1,99 mmol) (1-Ethoxyvinyl)tributylzinn, 91 mg (0,08 mmol) Tetrakis(triphenylphosphin)palladium, 130 mg (3,06 mmol) Lithiumchlorid und 0,161 ml (1,99 mmol) Pyridin hinzu. Anschließend wird 1 Stunde unter Rückfluß gekocht. Dann wird die Reaktionslösung über Celite filtriert. Das erhaltene Rohprodukt wird durch Säulenchromatographie an Kieselgel mit einem Gemisch aus Hexan/Ethylacetat gereinigt. Man erhält 583 mg 3,3-[1,2-Ethandiylbis(oxy)]-11β-[4-(1-ethoxyethenyl)phenyl]-17β-[3-[(tetrahydro-2H-pyran-2-yl)oxy]propyl]-5α,14β-estran-5,17α-diol und 143 mg 11β-[4-Acetylphenyl]-3,3-[1,2-ethandiylbis(oxy)]-17β-[3-[(tetrahydro-2*H*-pyran-2-yl)oxy]propyl]-5α,14β-estran-5,17α-diol.

### r) 11β-(4-Acetylphenyl)-17α-hydroxy-17β-(3-hydroxypropyl)-14β-estr-4-en-3-on

583 mg 3,3-[1,2-Ethandiylbis(oxy)]-11β-[4-(1-ethoxyethenyl)phenyl]-17β-[3-[(tetrahydro-2*H*-pyran-2-yl)oxy]propyl]-5α,14β-estran-5,17α-diol, sowie 143 mg 11β-[4-Acetylphenyl]-3,3-[1,2-ethandiylbis(oxy)]-17β-[3-[(tetrahydro-2H-pyran-2-yl)oxy]propyl]-5α,14β-estran-5,17α-diol werden in 25 ml Aceton gelöst. Man gibt 1,15 ml einer 4 molaren wäßrigen Salzsäurelösung hinzu und rührt 1 Stunde bei 50°C. Dann wird mit gesättigter Natriumhydrogencarbonatlösung versetzt und mit Methylenchlorid extrahiert. Die organische Phase wird mit gesättigter Natriumchloridlösung gewaschen und über Natriumsulfat getrocknet. Es wird im Vakuum eingeengt. Das Rohprodukt wird durch Säulenchromatographie an Kieselgel mit einem Gemisch aus Hexan/Ethylacetat gereinigt. Man erhält 420 mg 11β-(4-Acetylphenyl)-17α-hydroxy-17β-(3-hydroxypropyl)-14β-estr-4-en-3-on.

¹H-NMR (CDCl₃): δ= 7,90 d (J=9 Hz, 2H, Ar); 7,54 d (J=9 Hz, 2H, Ar); 5,83 sbr (1H, H-4); 3,60-3,77 m (2H, CH₂OH); 3,45 ddbr (J=9, 5 Hz, 1H, H-11); 2,60 s (3H, OAc); 0,72 s (3H, H-18)

### Beispiel 2

### 17α-Hydroxy-17β-(3-hydroxypropyl)-11β-[4-(3-pyridinyl)phenyl]-14β-estr-4-en-3-on

### a) 3,3-1,2-[Ethandiylbis(oxy)]-11β-[4-(3-pyridinyl)phenyl]-17β-[3[(tetrahydro-2H-pyran-2-yl)oxy]-propyl]-5α,14β-estran-5,17α-diol

1,08 g 3,3-[1,2-Ethandiylbis(oxy)]-17β-[3-[(tetrahydro-2*H*-pyran-2-yl)oxy]propyl]-11β-[4-[[(trifluor methyl)sulfonyl]oxy]phenyl]-5α,14β-estran-5,17α-diol werden in 15 ml Toluol und 6,5 ml Ethanol gelöst. Man addiert 250 mg Diethyl(3-pyridinyl)boran, 90 mg Tetrakis(triphenylphosphin)palladium, 132 mg Lithiumchlorid und 2 ml einer 2 molaren Natriumcarbonatlösung und kocht 1 Stunde unter Rückfluß. Anschließend wird mit Wasser verdünnt und dann mit Ethylacetat extrahiert. Die organische Phase wird mit gesättigter wäßriger Natriumchloridlösung gewaschen, über Natriumsulfat getrocknet, filtriert und am Vakuum eingeengt. Man erhält nach Säulenchromatographie an Kieselgel mit einem Gemisch aus Hexn/Ethylacetat 774 mg 3,3-1,2-[Ethandiylbis(oxy)]-11β-[4-(3-pyridinyl)phenyl]-17β-[3-[(tetrahydro-2*H*-pyran-2-yl)oxy]-propyl]-5α,14β-estran-5,17α-diol.

### b) 17α-Hydroxy-17β-(3-hydroxypropyl)-11β-[4-(3-pyridinyl)phenyl]-14β-estr-4-en-3-on

Wie unter Beispiel 1 r) beschrieben werden aus 774 mg 3,3-[1,2-Ethandiylbis(oxy)]-11β-[4-(3-pyridinyl)phenyl]-17β-[3-[(tetrahydro-2*H*-pyran-2-yl)oxy]-propyl]-5α,14β-estran-5,17α-diol in 25 ml Aceton und 1,5 ml 4 molarer wäßriger Salzsäure 476 mg 17α-Hydroxy-17β-(3-hydroxypropyl)-11β-[4(3-pyridinyl)phenyl]-14β-estr-4-en-3-on dargestellt.

¹H-NMR (CDCl₃): δ= 8,87 sbr (1H, Py); 8,58 dbr (J=4.5 Hz, 1H, Py); 7,87 dtr (J=7.5; 1 Hz, 1H, Py); 7,53 m (4H, Ar); 7,37 dd (J=7.5, 4 Hz, 1H, Py); 5,83 sbr (1H, H-4); 3,60-3,78 m (2H, CH₂OH); 3,45 ddbr (7.5, 5, 1H, H-11); 0,77 s (3H, H-18)

### Beispiel 3

### 17β-Hydroxy-11β-(4-methoxyphenyl)-17α-methylestra-4,14-dien-3-on

### a) 3,3-[1,2-Ethandiylbis(oxy)]-11β-(4-methoxyphenyl)-17α-methylestra-5,14-dien-17β-ol

2,46 g wasserfreies Certrichlorid werden in 20 ml absolutem Tetrahydrofuran suspendiert. Man rührt 2 Stunden bei Raumtemperatur, kühlt dann auf 0°C und versetzt mit 3,3 ml einer 3 molaren Lösung von Methylmagnesiumchlorid in Tetrahydrofuran. Anschließend läßt man anderthalb Stunden bei 0°C rühren und tropft dann eine Lösung von 1 g 3,3-[1,2-Ethandiylbis(oxy)]-11β-[4-methoxyphenyl)estra-5,14-dien-17-on in 10 ml absolutem Tetrahydrofuran hinzu. Man läßt weitere 1,5 Stunden bei 0°C rühren. Dann wird die Reaktionslösung auf gesättigte Ammoniumchloridlösung gegossen. Man extrahiert mit Ethylacetat, wäscht die organische Phase mit gesättigter Natriumchloridlösung und trocknet über Natriumsulfat. Das Rohprodukt wird durch Säulenchromatographie an Kieselgel mit einem Gemisch aus Hexan und Ethylacetat gereinigt. Man erhält 920 mg 3,3-[1,2-Ethandiylbis(oxy)]-11β-(4-methoxyphenyl)-17α-methylestra-5,14-dien-17β-ol als weißen Schaum.

¹H-NMR (CDCl₃): δ= 7,33 d (J=9 Hz, 2H, Ar); 6,81 d (J=9Hz, 2H, Ar); 5,59 m (1H, H-15); 3,88-4,00 m (4H, Ketal); 3,80 s (3H, OMe); 3,42 m (3H, Methyl); 0,70 s (3H, H-18)

### b) 17β-Hydroxy-11β-(4-methoxyphenyl)-17α-methylestra-4,14-dien-3-on

Wie unter Beispiel 1 r) beschrieben werden aus 910 mg 3,3-[1,2-Ethandiylbis(oxy)]-11β-(4-methoxyphenyl)-17α-methylestra-5,14-dien-17β-ol in 25 ml Aceton und 1,5 ml 4 molarer wäßriger Salzsäure 820 mg 17β-Hydroxy-11β-(4-methoxyphenyl)-17α-methylestra-4,14-dien-3-on dargestellt.

¹H-NMR (CDCl₃): δ= 7,49 d (J=9 Hz, 2H, Ar); 6,85 d (J=9 Hz, 2H, Ar); 5,88 sbr (1H, H-4); 5,28 m (1H, H-15); 3,80 s (3H, Ome); 3,37 ddbr (J=7, 5 Hz, 1H, H-11); 1,23 s (3H, Methyl); 0,80 s (3H, H-18)

[α]²⁰_{D}=+106,2° (CHCl₃; c=0,563)
Fp=186°C

### Beispiel 4

### 11β-[4-(3-Furanyl)phenyl]-17β-hydroxy-17α-methylestra-4,14-dien-3-on

### a) 3,3-[1,2-Ethandiylbis(oxy)]-11β-(4-hydroxyphenyl)-17α-methylestra-5,14-dien-17β-ol

Wie unter Beispiel 1o) beschrieben werden 3,7 g 3,3-[1,2-Ethandiylbis(oxy)]-11β-(4-methoxyphenyl)-17α-methyl-5,14-dien-17β-ol und 2,37 g Natriummethanthiolat in 50 ml Dimethylformamid umgesetzt. Nach Aufarbeitung wird das Rohprodukt durch Säulenchromatographie an Kieselgel mit einem Gemisch aus Hexan/Ethylacetat gereinigt. Man erhält 3,57 g 3,3-[1,2-Ethandiylbis(oxy)]-11β-(4-hydroxyphenyl)-17α-methylestra-5,14-dien-17β-ol als weißen Schaum.

¹H-NMR (CDCl₃): δ= 7,25 d (J=9 Hz, 2H, Ar); 6,70 d (J=9 Hz, 2H, Ar); 5,58 sbr (1H, H-5); 5,21 m (1H, H-15); 3,85-4,00 m (4H, Ketal); 3,42 m (3H, H-11); 1,22 s (3H, C-20); 0,69 s (3H, C-18)

### b) 3,3-[1,2-Ethandiylbis(oxy)]-17α-methyl-11β-[4-[[(trifluormethyl)sulfonyl]oxy]phenyl]estra-5,14-dien-17β-ol

Wie unter Beispiel 1p) beschrieben werden 3,57 g 3,3-[1,2-Ethandiylbis(oxy)]-11β-(4-hydroxyphenyl)-17α-methylestra-5,14-dien-17β-ol, 1,84 ml Trifluormethansulfonsäureanhydrid und 5,16 g 4-Dimethylaminopyridin in 100 ml absolutem Methylenchlorid umgesetzt. Man erhält nach Säulenchromatographie an Kieselgel mit einem Gemisch aus Hexan/Ethylacetat 3,7 g 3,3-[1,2-Ethandiylbis(oxy)]-17α-methyl-11β-[4-[[(trifluormethyl)sulfonyl]oxy]phenyl]estra-5,14-dien-17β-ol als weißen Schaum.

¹H-NMR (CDCl₃): δ= 7,50 d (J=8 Hz,2H,Ar); 7,18 d (J=8 Hz,2H,Ar); 5,60 m (1H, H-5); 5,24 m (1H,H-15); 3,85-4,00 m (4H, Ketal); 3,50 ddbr (J=5, 6 Hz,1H,H-11); 1,22 s (3H, C-20); 0,62 s (3H, C-18);

### c) 3,3-[1,2-Ethandiylbis(oxy)]-11β-[4(3-furanyl)phenyl]-17α-methylestra-5,14-dien-17β-ol

Darstellung von (3-Furanyl)tributylstannan: 69,5 ml einer 1,6 molaren Lösung von n-Butyllithium in Hexan werden mit 60 ml absolutem Tetrahydrofuran gemischt. Man kühlt auf -60°C und tropft 10 ml 3-Bromfuran mit einer solchen Geschwindigkeit hinzu, daß die Innentemperatur nicht -50°C überschreitet. Nach vollständiger Zugabe wird 15 Minuten bei -60°C nachgerührt und dann werden 33,2 ml Tributylzinnchlorid hinzugetropft, wobei die Innentemperatur auch hierbei -50°C nicht übersteigen darf. Nach vollständiger Zugabe läßt man die Reaktionslösung auf -10°C kommen und rührt eine Stunde bei dieser Temperatur nach. Anschließend wird vorsichtig mit Wasser gequencht. Man extrahiert die wäßrige Phase mit Essigester. Die organische Phase wird mit gesättigter Natriumchloridlösung gewaschen und über Natriumsulfat getrocknet. Man filtriert ab und zieht das Lösungsmittel am Vakuum ab. Das erhaltene Rohprodukt wird durch Destillation am Hochvakuum gereinigt. Man erhält 30,14 g (3-Furanyl)tributylstannan als leicht gelbes Öl (Siedepunkt= 100-104°C bei 0,09 Torr).
Kupplung: 1,8 g 3,3-[1,2-Ethandiylbis(oxy)]-17α-methyl-11β-[4-[[(trifluormethyl)sulfonyl]oxy]phenyl]estra-5,14-dien-17β-ol, 1,51 g (3-Furanyl)tributylstannan, 185 mg Tetrakis(triphenylphosphin)palladium, 275 mg Lithiumchlorid und 0,34 ml Pyridin werden analog zu Beispiel 1q) in 25 ml Dioxan umgesetzt. Das Rohprodukt wird durch Säulenchromatographie an Kieselgel mit einem Gemisch aus Hexan/Ethylacetat gereinigt. Man erhält 1,15 g 3,3-[1,2-Ethandiylbis(oxy)]-11β-[4-(3-furanyl)phenyl]-17α-methylestra-5,14-dien-17β-ol als weißen Schaum.

¹H-NMR (CDCl₃): δ= 7,70 dbr (J=1.3 Hz,1H,Fu-2); 7,48 dd (J=1.8, 1.3 Hz,1H,Fu-5); 7,40 m (4H, Ar); 6,70 dbr (J=1.8,1H,Fu-4); 5,60 m (1H,H-6); 5,20 m (1H,H-15); 3,90-4,00 m (4H,Ketal); 3,50 ddbr (J=5, 6 Hz,1H,H-11); 1,20 s (3H,C-20); 0,70 s (3H,C-18)

### d) 11β-[4-(3-Furanyl)phenyl]-17β-hydroxy-17α-methylestra-4,14-dien-3-on

1,15 g 3,3-[1,2-Ethandiylbis(oxy)]-11β-[4-(3-furanyl)phenyl]-17α-methylestra-5,14-dien-17β-ol und 2,9 ml 4 normale wäßrige Salzsäurelösung in 60 ml Aceton werden wie unter Beispiel 1r) beschrieben umgesetzt. Man erhält nach Säulenchromatographie an Kieselgel mit einem Gemisch aus Hexan/Ethylacetat und Umkristallisation aus einem Gemisch aus Diisopropylether/Hexan 794 mg 11β-[4-(3-Furanyl)phenyl]-17β-hydroxy-17α-methylestra-4,14-dien-3-on als weiße Kristalle.

¹H-NMR (CDCl₃): δ= 7,73 dbr (1.3 Hz,1H,Fu-2); 7,45-7,53 m (5H,Ar und Fu-5); 6,70 dbr (J=1.8 Hz,1H,Fu-4); 5,90 m (1H,H-4); 5,30 m (1H,H-15); 3,41 ddbr (J=5, 6 Hz,1H,H-11); 1,22 s (3H,C-20); 0,80 s (3H,C-18)

[α]²⁰_{D}=+153° (CHCl₃; c=0,510)
Fp=198,9°C

### Beispiel 5:

### 11β-(4-Acetylphenyl)17β-hydroxy-17α-methylestra-4,14-dien-3-on

### a) 3,3-[1,2-Ethandiylbis(oxy)]-11β-[4(1-ethoxyethenyl)phenyl]-17α-methylestra-5,14-dien-17β-ol

Wie unter Beispiel 1q) beschrieben werden 1,8 g 3,3-[1,2-Ethandiylbis(oxy)]-17α-methyl-11β-4-[[(trifluormethyl)sulfonyl]oxy]phenyl]estra-5,14-dien-17β-ol, 1,43 ml (1-Ethoxyvinyl)tributylzinn, 185 mg Tetrakis(triphenylphosphin)palladium, 275 mg Lithiumchlorid und 0,34 ml Pyridin in 25 ml Dioxan umgesetzt. Man erhält 1,31 g 3,3-[1,2-Ethandiylbis(oxy)]-11β-[4-(1-ethoxyethenyl)phenyl]-17α-methylestra-5,14-dien-17β-ol, welches ohne Reinigung in die nächste Stufe eingesetzt wird.

### b) 11β-(4-Acetylphenyl)17β-hydroxy-17α-methylestra-4,14-dien-3-on

Aus 1,31 g 3,3-[1,2-Ethandiylbis(oxy)]-11β-[4-(1-ethoxyethenyl)phenyl]-17 α-methylestra-5,14-dien-17β-ol und 3,3 ml 4 normaler wäßriger Salzsäure in 65 ml Aceton werden analog zu Beispiel 1r) nach Säulenchromatographie an Kieselgel mit einem Gemisch aus Hexan/Ethylacetat und Kristallisation aus Diisopropylether/Aceton 860 mg 11β-(4-Acetylphenyl)-17β-hydroxy-17α-methylestra-4,14-dien-3-on als weiße Kristalle erhalten.

¹H-NMR (CDCl₃): δ= 7,92 d (J=8 Hz,2H,Ar); 7,61 d (J=8 Hz,2H,Ar); 5,90 m (1H,H-4); 5,30 m (1H,H-14); 3,49 ddbr (J=5, 6 Hz,1H,H-11); 2,60 s (3H, Acetyl); 1,23 s (3H,C-20); 0,73 s (3H,C-18)

[α]²⁰_{D}=+145,3° (CHCl₃; c=0,525)
Fp=195,1°C

### Beispiel 6:

### 17β-Hydroxy-17α-methyl-11β-[4-(3-thienyl)phenyl]estra-4,14-dien-3-on

### a) 3,3-[1,2-Ethandiylbis(oxy)]-17α-methyl-11β-[4-(3-thienyl)phenyl]estra-5,14-dien-17β-ol

Darstellung von (3-Thienyl)tributylstannan:
Aus 15 ml 3-Bromthiophen und 100 ml einer 1,6 molaren Lösung von Butyllithium in Hexan sowie 38,94 ml Tributylzinnchlorid werden in 100 ml Tetrahydrofuran analog zu Beispiel 4c) 52,1 g (3-Thienyl)tributylstannan hergestellt
Aus 920 mg 3,3-[1,2-Ethandiylbis(oxy)]-17α-methyl-11β-[4-[[(trifluormethyl)sulfonyl]-oxy]phenyl]estra-5,14-dien-17β-ol, 805 mg (3-Thienyl)tributylstannan, 94 mg Tetrakis(triphenylphosphin)palladium, 141 mg Lithiumchlorid und 0,18 ml Pyridin werden in 25 ml Dioxan analog zu Beispiel 1q) nach Säulenchromatographie an Kieselgel mit einem Gemisch aus Hexan/Ethylacetat 420 mg 3,3-[1,2-Ethandiylbis(oxy)]-17α-methyl- 11β-[4-(3-thienyl)phenyl]estra-5,14-dien-17β-ol als weißer Schaum erhalten.

### b) 17β-Hydroxy-17α-methyl-11β-[4-(3-thienyl)phenyl]estra-4,14-dien-3-on

Aus 420 mg 3,3-[1,2-Ethandiylbis(oxy)]-17α-methyl-11β-[4-(3-thienyl)phenyl]estra-5,14-dien-17β-ol und 1,25 ml 4 nomaler wäßriger Salzsäure in 25 ml Aceton werden analog zu Beispiel 1r) nach Chromatographie an Kieselgel mit einem Gemisch aus Hexan/Ethylacetat und Umkristallisation aus Diisopropylether 250 mg 17β-Hydroxy-17α-methyl-11β-[4-(3-thienyl)phenyl]estra-4,14-dien-3-on als weiße Kristalle erhalten.

¹H-NMR (CDCl₃): δ= 7,52 m (4H,Ar); 7,46 dd (J=4.5, 2.5 Hz,1H,Th-5); 7,40 dbr (J=2.5 Hz,1H,Th-2); 7,26 dbr (J=4.5 Hz,1H,Th-4); 5,90 sbr (1H,H-4); 5,30 m (1H,H-15); 3,45 ddbr (J=5, 6 Hz,1H,H-11); 1,25 s (3H, C-20); 0,80 s (3H,C-18)

[α]²⁰_{D}=+170,5° (CHCl₃); c=0,520)
Fp=182,0°C

### Beispiel 7:

### 17β-Hydroxy-11β-(4-methoxyphenyl)-17α-methylestra-4,15-dien-3-on

### a) 3,3-[1,2-Ethandiylbis(oxy)]-11β-(4-methoxyphenyl)-17α-methylestra-5,15-dien-17β-ol

30 ml einer 1,6 molaren Lösung von Methyllithium in Diethylether werden mit 70 ml absolutem Tetrahydrofuran verdünnt. Man kühlt auf 0°C und tropft langsam eine Lösung von 4 g 3,3-[1,2-Ethandiylbis(oxy)]-11β-(4-methoxyphenyl)estra-5,15-dien-17-on in 30 ml Tetrahydrofuran hinzu. Anschließend wird 1 Stunde bei 0°C nachgerührt. Dann wird die Reaktionslösung auf gesättigte Natriumhydrogencarbonatlösung gegossen. Man extrahiert mehrmals mit Ethylacetat, wäscht sie vereinigten organischen Phasen mit gesättigter wäßriger Natriumchloridlösung, trocknet über Natriumsulfat und engt am Vakuum ein. Man erhält 4,15 g 3,3-[1,2-Ethandiylbis(oxy)]-11β-(4-methoxyphenyl)-17α-methylestra-5,15-dien-17β-ol, welches ohne Reinigung in die Folgestufen eingesetzt wird.

### b) 17β-Hydroxy-11β-(4-methoxyphenyl)-17α-methylestra-4,15-dien-3-o n

Wie unter Beispiel 1r) beschrieben werden 840 mg 3,3-[1,2-Ethandiylbis(oxy)]-11β-(4-methoxyphenyl]-17α-methylestra-5,15-dien-17β-ol und 2,5 ml 4 normaler wäßriger Salzsäure in 50 ml Aceton umgesetzt Man erhält nach Säulenchromatographie an Kieselgel mit einem Gemisch aus Hexan/Ethylacetat, sowie Umkristallisation aus Diisopropylether 510 mg 17β-Hydroxy-11β-(4-methoxyphenyl)-17α-methylestra-4,15-dien-3-on als weiße Kristalle.

¹H-NMR (CDCl₃): δ= 7,32 d (J=8 Hz,2H,Ar); 6,81 d (J=8 Hz,2H,Ar); 5,87 m (2H,H-4,H-16); 5,60 dd (J=5, 2.5 Hz,1H,H-15); 3,80 s (3H,OMe); 3,40 ddbr (J=5, 6 Hz,1H,H-11); 1,17 s (3H, C-20); 0,72 s (3H, C-18)

[α]²⁰_{D}=+51,3° (CHCl₃; C=0,520)
Fp=191,5°C

### Beispiel 8

### 17β-Hydroxy-11β-(4-methoxyphenyl)-17α-(1-propinyl)estra-4,15-dien-3-on

### a) 3,3-[1,2-Ethandiylbis(oxy)]-11β-(4-methoxyphenyl)-17α-(1-propinyl)estra-5,15-dien-17β-ol

100 ml absolutes Tetrahydrofuran werden bei 0°C 30 Minuten mit Propingas gesättigt. Anschließend tropft man 12,5 ml einer 1,6 molaren Lösung von Butyllithium in n-Hexan hinzu und läßt 30 Minuten nachrühren. Danach wird eine Lösung von 840 mg 3,3-[1,2-Ethandiylbis(oxy)]-11β-(4-methoxyphenyl)-17α-methylestra-5,15-dien-17β-ol in 20 ml absolutem Tetrahydrofuran hinzugetropft. Man läßt weitere 1,5 Stunden bei 0°C nachrühren, quencht dann mit gesättigter wäßriger Ammoniumchloridlösung und extrahiert mit Ethylacetat Die organische Phase wird mit gesättigter wäßriger Natriumchloridlösung gewaschen und über Natriumsulfat getrocknet. Das Lösungsmittel wird am Vakuum abgezogen und man erhält 920 mg 3,3-[1,2-Ethandiylbis(oxy)]-11β-(4methoxyphenyl)-17α-(1-propinyl)estra-5,15-dien-17β-ol, welches ohne Reinigung in die Folgestufe eingesetzt wird.

¹H-NMR (CDCl₃): δ= 7,24 d (J=8 Hz,2H,Ar); 6,79 d (J=8 Hz,2H,Ar); 5,98 dbr (J=5 Hz,1H,H-16); 5,66 dd (J=5, 2.5 Hz,1H,H-15); 5,52 m (1H, H-6); 3,90-4,00 m (4H,Ketal); 3,80 s (3H,OMe); 3,50 ddbr (J=5, 7 Hz,1H,H-11); 1,90 s (3H,Propin); 0,69 s (H,C-18)

### b) 17β-Hydroxy-11β-(4-methoxyphenyl)-17α-(1-Propinyl)estar-4,15-dien-3-on

Wie unter Beispiel 1r) beschrieben werden 920 mg 3,3-[1,2-Ethandiylbis(oxy)]-11β-(4-methoxyphenyl)-17α-(1-propinyl)estra-5,15-dien-17β-ol und 2,5 ml 4 normale wäßrige Salzsäure in 50 ml Aceton umgesetzt. Man erhält nach Säulenchromatographie an Kieselgel mit einem Gemisch aus Hexan/Ethylacetat und Umkristallisation aus Diisopropylether 691 mg 17β-Hydroxy-11β-(4-methoxyphenyl)-17α-(1-Propinyl)estra-4,15-dien-3-on als weiße Kristalle.

¹H-NMR (CDCl₃): δ= 7,31 d (J=8 Hz,2H,Ar); 6,81 d (J=8 Hz,2H,Ar); 6,00 dbr (J=5 Hz,1H,H-16); 5,87 sbr (1H,H-4); 5,69 dd (J=5, 2.5 Hz,1H,H-15); 3,80 s (3H,OMe); 3,42 ddbr (J=5, 7 Hz,1H,H-11); 1,90 s (3H, Propin); 0,73 s (3H,C-18)

[α]²⁰_{D}=+170° (CHCl₃; c=0,510)
Fp=191,5°C

### Beispiel 9

### (Z)-17β-Hydroxy-17α-(3-hydroxy-1-propenyl)-11β-(4-methoxyphenyl)estra-4,15-dien-3-on

### a) 3,3-[1,2-Ethandiylbis(oxy)]-11β-(4-methoxyphenyl)-17α-[3-[(tetrahydro-2H-pyran-2-yl)oxy] 1-propinyl]estra-5,15-dien-17β-ol

840 mg 3,3-[1,2-Ethandiylbis(oxy)]-11β-(4-methoxyphenyl]-17α-methylestra-5,15-dien-17β-ol, 2,81 ml 3-[(Tetrahydro-2H-pyran-2-yl)oxy]-1-propin und 12,5 ml einer 1,6 molaren Lösung von Butyllithium in Hexan werden in 100 ml absolutem Tetrahydrofuran wie unter Beispiel 1m) beschrieben umgesetzt. Man erhält nach Säulenchromatographie an Kieselgel mit einem Gemisch aus Hexan/Ethylacetat 1,05 g 3,3-[1,2-Ethandiylbis(oxy)]-11β-(4-methoxyphenyl)-17α-[3-[(tetrahydro-2H-pyran-2-yl)oxy]-1-propinyl]estra-5,15-dien-17β-ol als weißen Schaum.

¹H-NMR (CDCl₃): δ= (Gemisch der isomeren THP-Ether
7,21 d (J=8 Hz,2H,Ar); 6,79 d (J=8 Hz,2H,Ar); 6,00 dbr (J=6 Hz,1H,H-16); 5,67 dd (J=6, 2.5 Hz,1H,H-15); 5,55 m (1H,H-6); 4,62 m (1H,THP); 4,38 m (2H,CH₂OTHP); 3,80-4,00 m (4H,Ketal); 3,80 s (3H,OMe); 3,50-3,60 m (2H,THP); 0,70 (3H,C-18)

### b) 17β-Hydroxy-11β-(4-methoxyphenyl)-17α-[3-[(tetrahydro-2H-pyran-2-yl)oxy]-1-propinyl]-estra-4,15-dien-3-on

1,05 g 3,3-[1,2-Ethandiylbis(oxy)]-11β-(4-methoxyphenyl)-17α-[3-[(tetrahydro-2H-pyran-2-yl)oxy]-1-propinyl]estra-5,15-dien-17β-ol und 2,5 ml 4 normale wäßrige Salzsäure werden in 50 ml Aceton wie unter Beispiel 1r) beschrieben umgesetzt. Man erhält nach Säulenchromatographie an Kieselgel mit einem Gemisch aus Hexan/Ethylacetat 690 mg 17β-Hydroxy-11β-(4-methoxyphenyl)-17α-[3[(tetrahydro-2H-pyran-2-yl)oxy]-1-propinyl]estra-4,15-dien-3-on als weißen Schaum.

¹H-NMR (CDCl₃): δ= 7,31 d (J=8 Hz,2H,Ar); 6,82 d (J=8 Hz,2H,Ar); 6,03 dbr (J=6 Hz,1H,H-16); 5,88 sbr (1H,H-4); 5,70 dd (J=6, 2.5 Hz,1H,H-15); 4,38 m (2H,CH₂OH); 3,80 s (3H,OMe); 3,41 ddbr (J=5, 6 Hz,1H,H-11); 0,75 s (3H,C-18)

### c) (Z)-17β-Hydroxy-17α-(3-hydroxy-1-propenyl)-11β-(4-methoxyphenyl)estra-4,15-dien-3-on

690 mg 17β-Hydroxy-11β-(4-methoxyphenyl)-17α-[3-[(tetrahydro-2H-pyran-2-yl)oxy]-1-propinyl]estra-4,15-dien-3-on werden in 10 ml Tetrahydrofuran gelöst. Man addiert 0,69 ml Pyridin und 69 mg Palladium (10% auf Bariumsulfat). Anschließend wird 1 Stunde unter Wasserstoff hydriert. Danach wird die Reaktionslösung über Celite filtriert und am Vakuum eingeengt. Säulenchromatographie an Kieselgel mit einem Gemisch aus Hexan/Ethylacetat ergibt 500 mg (Z)-17β-Hydroxy-17α-(3-hydroxy-1-propenyl)-11β-(4-methoxyphenyl)-estra-4,15-dien-3-on als weißen Schaum.

¹H-NMR (CDCl₃): δ= 7,30 d (J=8 Hz,2H,Ar); 6,80 d (J=8 Hz,2H,Ar); 5,99 d (J=6 Hz,1H,H-16); 5,89 sbr (1H,H-4); 5,71 ddd (J=12, 5.5, 5 Hz,1H,HC=); 5,69 dd (J=6, 2.5 Hz,1H,H-15); 5,54 dbr (J=12 Hz,1H,HC=); 4,25 m (2H,CH₂OH); 3,80 s (3H,OMe); 3,38 ddbr (J=5, 7 Hz,1H,H-11); 0,80 s (3H,C-20)

### Beispiel 10

### 11β-(4-Acetylphenyl)-17β-hydroxy-17α-methylestra-4,15-dien-3-on

### a) 3,3-[1,2-Ethandiylbis(oxy)]-11β-(4-hydroxyphenyl)-17α-methylestra-5,15-dien-17β-ol

Wie unter Beispiel 1o) beschrieben werden 4,15 g 3,3-[1,2-Ethandiylbis(oxy)]-11β-(4-methoxyphenyl)-17α-methylestra-5,15-dien-17β-ol und 2,66 g Natriummethanthiolat in 50 ml Dimethylformamid umgesetzt. Man erhält nach Säulenchromatographie an Kieselgel mit einem Gemisch aus Hexan/Ethylacetat 3,5 g 3,3-[1,2-Ethandiylbis(oxy)]-11β-(4-hydroxyphenyl)-17α-methylestra-5,15-dien-17β-ol als weißen Schaum.

¹H-NMR (CDCl₃): δ= 7,15 d (J=8 Hz,2H,Ar); 6,70 d (J=8 Hz,2H,Ar); 5,84 dbr (J=6 Hz,1H,H-16); 5,53 dd (J=6, 2.5 Hz,1H,H-15); 3,90-4,05 m (4H,Ketal); 3,40 ddbr (J=5, 7 Hz,1H,H-11); 3,35 sbr (1H,Phenol); 1,12 s (3H,C-20); 0,70 s (3H,C-18)

### b) 3,3-[1,2-Ethandiylbis(oxy)]-17α-methyl-11β-[4[[(1,1,2,2,3,3,4,4,4-nonafluorbutyl)sulfonyl]oxy]phenyl]estra-5,15-dien-17β-ol

Zu 3,22 g 3,3-[1,2-Ethandiylbis(oxy)]-11β-(4-hydroxyphenyl)-17α-methyl-5,15-dien-17β-ol in 140 ml absolutem Tetrahydrofuran werden bei 0°C 5,5 ml einer 1,6 molaren Lösung von Butyllithium in Hexan hinzugetropft. Man läßt 30 Minuten bei 0°C nachrühren und tropft dann 3,04 ml Nonafluorbutansulfonylfluorid hinzu. Anschließend läßt man über 2 Stunden auf Raumtemperatur kommen und gießt dann die Reaktionslösung auf eiskalte gesättigte Natriumhydrogencarbonatlösung. Man läßt eine weitere Stunde nachrühren und extrahiert anschließend mit Ethylacetat. Die organische Phase wird mit gesättigter wäßriger Natriumchloridlösung gewaschen und über Natriumsulfat getrocknet. Man engt am Vakuum ein und reinigt das Rohprodukt durch Säulenchromatographie an Kieselgel mit einem Gemisch aus Hexan/Ethylacetat. Man erhält 4,4 g 3,3-[1,2-Ethandiylbis(oxy)]-17α-methyl-11β-[4-[[(1,1,2,2,3,3,4,4,4-nonafluorbutyl)sulfonyl]oxy]phenyl]estra-5,15-dien-17β-ol als weißen Schaum.

¹H-NMR (CDCl₃): δ= 7,43 d (J=8 Hz,2H,Ar); 7,17 d (J=8 Hz,2H,Ar); 5,85 dbr (J=6 Hz,1H,H-16); 5,57 dd (J=6, 2.5 Hz,1H,H-15); 3,90-4,05 m (4H,Ketal); 3,55 ddbr (J=5, 7 Hz,1H,H-11); 1,15 s (3H,C-20); 0,62 s (3H,C-18)

### c) 3,3-[1,2-Ethandiylbis(oxy)]-11β-[4-(1-ethoxyethenyl)phenyl]-17α-methylestra-5,15-dien-17β-ol

Wie unter Beispiel 1q) beschrieben werden 2,26 g 3,3-[1,2-Ethandiylbis(oxy)]-17α-methyl-11β-[4-[[(1,1,2,2,3,3,4,4,4-nonafluorbutyl)sulfonyl]oxy]phenyl]estra-5,15-dien-17β-ol, 1,43 ml (1-Ethoxyvinyl)tributylzinn, 185 mg Tetrakis(triphenylphosphin)palladium, 275 mg Lithiumchlorid und 0,34 ml Pyridin in 25 ml absolutem Dioxan umgesetzt. Das Rohprodukt wird ohne Aufreinigung in die nächste Stufe eingesetzt.

### d) 11β-(4-Acetylphenyl)-17β-hydroxy-17α-methylestra-4,15-dien-3-on

Wie unter Beispiel 1r) beschrieben werden 1,37 g 3,3-[1,2-Ethandiylbis(oxy)]-11β-[4-(1-ethoxyethenyl)phenyl]-17α-methylestra-5,15-dien-17β-ol und 3 ml 4 normale wäßrige Salzsäure in 75 ml Aceton umgesetzt. Man erhält nach Säulenchromatographie an Kieselgel mit einem Gemisch aus Hexan/Ethylacetat und Umkristallisation aus Diisopropylether 700 mg 11β-(4-Acetylphenyl)-17β-hydroxy-17α-methylestra4,15-dien-3-on als weiße Kristalle.

¹H-NMR (CDCl₃): δ= 7,90 d (J=8 Hz,1H,Ar); 7,52 d (J=8 Hz,1H,Ar); 5,90 m (2H,H-4,H-16); 5,60 dd (J=6, 2.5 Hz,1H,H-15); 3,50 ddbr (J=5, 7 Hz,1H,H-11); 2,60 s (3H,Acetyl); 1,20 s (3H,C-20); 0,70 s (3H,C-18)

[α]²⁰_{D}=+89,5° (CHCl₃; c=0,505)
Fp=223,7°C

### Beispiel 11

### 11β-[4-(3-Furanyl)phenyl]-17β-hydroxy-17α-methylestra-4,15-dien-3-on

### a) 3,3-[1,2-Ethandiylbis(oxy)]-11β-[4-(3-furanyl)phenyl]-17α-methyalestr-5,15-dien-17β-ol

Wie unter Beispiel 1q) beschrieben werden 705 mg 3,3-[1,2-Ethandiylbis(oxy)]-17α-methyl-11β-[4-[[(1,1,2,2,3,3,4,4,4-nonafluorbutyl)sulfonyl]oxy]phenyl]estra-5,15-dien-17β-ol, 0,58 g (3-Furanyl)tributylstannan, 60 mg Tetrakis(triphenylphosphin)palladium, 85 mg Lithiumchlorid in 10 ml Dioxan umgesetzt. Man erhält nach Säulenchromatographie an Kieselgel mit einem Gemisch aus Hexan/Ethylacetat 320 mg 3,3-[1,2-Ethandiylbis(oxy)]-11β-[4(3-furanyl)phenyl]-17α-methylestra-5,15-dien-17β-ol als weißen Schaum.

¹H-NMR (CDCl₃): δ= 7,72 dbr (J=1.3 Hz,1H,Fu-2); 7,48 dd (J=1.8, 1.3 Hz,1H,Fu-5); 7,35-7,43 m (4H,Ar); 6,70 dbr (J=1.8 Hz,1H,Fu-4); 5,87 dbr (J=6 Hz,1H,H-16); 5,52-5,60 m (2H,H-15,H-6); 3,90-4,00 m (4H,Ketal); 3,53 ddbr (J=5, 7 Hz,1H-H-11); 1,28 s (3H,C-20); 0,70 s (3H,C-18)

### b) 11β-[4-(3-Furanyl)phenyl]-17β-hydroxy-17α-methylestra-4,15-dien-3-on

Wie unter Beispiel 1r) beschrieben werden 1,32 g 3,3-[1,2-Ethandiylbis(oxy)]-11β-[4-(3-furanyl)phenyl]-17α-methylestar-5,15-dien-17β-ol und 3 ml 4 normale wäßrige Salzsäure in 75 ml Aceton umgesetzt. Man erhält nach Säulenchromatographie an Kieselgel mit einem Gemisch aus Hexan/Ethylacetat sowie Umkristallisation aus Diisopropylether 894 mg 11β-[4-(3-Furanyl)phenyl]-17β-hydroxy-17α-methylestra-4,15-dien-3-on als weiße Kristalle.

¹H-NMR (CDCl₃): δ= 7,72 dbr (J=1.3 Hz,1H,Fu-2); 7,49 dd (1.8, 1.3 Hz,1H,Fu-5); 7,40-7,44 m (4H,Ar); 6,70 dbr (J=1.8 Hz,1H,Fu-4); 5,89 m (2H,H-4,H-16); 5,60 dd (J=6, 2.5 Hz,1H,H-15); 3,47 ddbr (J=5, 7 Hz,1H,H-11); 1,20 s (3H,C-20); 0,80 s (3H,C-18)

[α]²⁰_{D}=+111,4° (CHCl₃;c=0,515)
Fp=178,9°C

### Beispiel 12

### 17β-Hydroxy-17α-methyl-11β-[4-(3-pyridinyl)phenyl]estra-4,15-dien-3-on

### a) 3,3-[1,2-Ethandiylbis(oxy)]-17α-methyl-11β-[4-(3-pyridinyl)phenyl]estra-5,15-dien-17β-ol

Analog zu Beispiel 2a) werden 1,3 g 3,3-[1,2-Ethandiylbis(oxy)]-17α-methyl-11β-[4-[[(1,1,2,2,3,3,4,4,4-nonafluorbutyl)sulfonyl]oxy]phenyl]estra-5,15-dien-17β-ol, 310 mg Diethyl-(3-pyridinyl)boran, 110 mg Tetrakis(triphenylphosphin)palladium, 160 mg Lithiumchlorid und 2,4 ml einer 2 molaren wäßrigen Natriumcarbonatlösung in 15 ml Toluol und 7 ml Ethanol umgesetzt. Man erhält nach Säulenchromatographie an Kieselgel mit einem Gemisch aus Hexan/Ethylacetat 890 mg 3,3-[1,2-Ethandiylbis(oxy)]-17α-methyl-11β-[4-(3-pyridinyl)phenyl]estra-5,15-dien-17β-ol als weißen Schaum.

¹H-NMR (CDCl₃): δ= 8,87 sbr (1H,Py); 8,67 dbr (J=4.5 Hz,1H,Py); 7,90 dtr (7.5, 1 Hz,1H,Py); 7,68 m (1H,Ar); 7,45-7,55 m (3H,Ar); 7,35 dd (J=7.5, 4.5 Hz,1H,Py); 5,89 dbr (J=6 Hz,1H,H-16); 5,55-5,62 m (2H,H-6,H-15); 3,90-4,00 m (4H,Ketal); 3,60 ddbr (J=5, 7 Hz,1H,H-11); 1,90 s (3H,C-20); 0,73 s (3H,C-18)

### b) 17β-Hydroxy-17α-methyl-11β-[4-(3-pyridinyl)phenyl]estra-4,15-dien-3-on

Wie unter Beispiel 1 r) beschrieben werden 890 mg 3,3-[1,2-Ethandiylbis(oxy)]-17α-methyl-11β-[4-(3-pyridinyl)phenyl]estra-5,15-dien-17β-ol und 3,2 ml 4 normale wäßrige Salzsäure in 65 ml Aceton umgesetzt. Nach Säulenchromatographie an Kieselgel mit einem Gemisch aus Hexan/Ethylacetat und Umkristallisation aus einem Gemisch aus Diisopropylether/Ethylacetat werden 550 mg 17β-Hydroxy-17α-methyl-11β-[4-(3-pyridinyl)phenyl]-estra-4,15-dien-3-on als weiße Kristalle erhalten.

¹H-NMR (CDCl₃): δ= 8,88 sbr (1H,Py); 8,60 dbr (J=4.5 Hz,1H,Py); 7,90 dtr (7.5, 1 Hz,1H,Py); 7,70 m (1H,Ar); 7,50-7,60 m (3H,Ar); 7,36 dd (J=7.5, 4.5 Hz,1H,Py); 5,90 m (2H,H-4,H-16); 5,60 dd (J=6, 2.5 Hz,1H,H-15); 3,52 ddbr (J=5, 7 Hz,1H,H-11); 1,20 s (3H,C-20); 0,78 s (3H,C-18)

[α]²⁰_{D}=113,9° (CHCl₃;c=0,510)
Fp=158,6°C

### Beispiel 13

### 4'-[17β-Hydroxy-17α-methyl-3-oxoestra-4,15-dien-11β-yl][1,1'-biphenyl]-4-carbonitril

### a) 4'-[3,3-[1,2-Ethandiylbis(oxy)]-17β-hydroxy-17α-methylestra-5,15-dien-11β-yl][1,1'-biphenyl]-4-carbonitril

Analog zu Beispiel 2a) werden 2,4 g 3,3-[1,2-Ethandiylbis(oxy)]-17α-methyl-11β-[4-[[(1,1,2,2,3,3,4,4,4-nonafluorbutyl)sulfonyl]oxy]phenyl]estra-5,15-dien-17β-ol, 650 mg (4-Cyanphenyl)boronsäure, 200 mg Tetrakis(triphenylphosphin)palladium 290 mg Lithiumchlorid und 4,3 ml einer 2 molaren wäßrigen Natriumcarbonatlösung in 25 ml Toluol und 12 ml Ethanol umgesetzt Man erhält nach Säulenchromatographie an Kieselgel mit einer Mischung aus Hexan/Ethylacetat 1,63 g 4'-[3,3-[1,2-Ethandiylbis(oxy)]-17β-hydroxy-17α-methylestra-5,15-dien-11β-yl][1,1-biphenyl]-4-carbonitril als weißen Schaum.

¹H-NMR (CDCl₃): δ= 7,70-7,73 m (4H,Ar); 7,45-7,55 m (4H,Ar); 7,88 dbr (J=6 Hz,1H,H-16); 5,55-5,60 m (2H,H-6,H-15); 3,90-4,00 m (4H,Ketal); 3,58 ddbr (J=5, 7 Hz,1H,H-11); 1,20 s (3H,C-20); 0,70 s (3H,C-18)

### b) 4'-[17β-Hydroxy-17α-methyl-3-oxoestra-4,15-dien-11β-yl][1,1-biphenyl]-4-carbonitril

Analog zu Beispiel 1r) werden 500 mg 4'-[3,3-[1,2-Ethandiylbis(oxy)]-17β-hydroxy-17α-methylestra-5,15-dien-11β-yl][1,1'-biphenyl]-4-carbonitril und 1,25 ml 4 normale wäßrige Salzsäure in 25 ml Aceton umgesetzt. Nach Säulenchromatographie an Kieselgel mit einem Gemisch aus Hexan/Ethylacetat und Umkristalisation aus Diisopropylether werden 380 mg 4'-[17β-Hydroxy-17α-methyl-3-oxoestra-4,15-dien-11β-yl][1,1'-biphenyl]-4-carbonitril als weiße Kristalle erhalten.

¹H-NMR (CDCl₃): δ= 7,70-7,74 m (4H-Ar); 7,50-7,55 m (4H,Ar); 5,90 m (2H,H-4,H-16); 5,60 dd (J=6, 2.5 Hz,1H,H-15); 3,62 ddbr (5, 7 Hz,1H,H-11); 1,20 s (3H,C-20); 0,78 s (3H,C-18)

[α]²⁰_{D}=150,6° (CHCl₃;c=0,515)
Fp=205,5°C

### Beispiel 14

### 4'-17β-Methoxy-17α-methyl-3-oxoestra-4,15-dien-11β-yl][1,1'-biphenyl]-4-carbonitril

### a) 4'-[3,3-[1,2-Ethandiylbis(oxy)]-17β-methoxy-17α-methylestra-5,15-dien-11β-yl][1,1'-biphenyl]-4-carbonitril

Zu einer Suspension von 120 mg Natriumhydrid (60%ig in Parafinöl) in 5 ml absolutem Tetrahydrofuran werden bei Raumtemperatur 500 mg 4'-[3,3-[1,2-Ethandiylbis(oxy)]-17β-hydroxy-17α-methylestra-5,15-dien-11β-yl][1,1'-biphenyl]-4-carbonitril, gelöst in 15 ml absolutem Tetrahydrofuran gegeben. Danach addiert man 0,38 ml Iodmethan und kocht 2 Stunden unter Rückfluß. Anschließend wird vorsichtig mit gesättigter Natriumhydrogencarbonatlösung gequencht und mit Ethylacetat extrahiert. Die organische Phase wird mit gesättigter wäßriger Natriumchloridlösung gewaschen, über Natriumsulfat getrocknet, filtriert und am Vakuum einrotiert. Man erhält nach Säulenchromatographie an Kieselgel mit einem Gemisch aus Hexan/Ethylacetat 260 mg 4'-[3,3-[1,2-Ethandiylbis(oxy)]-17β-methoxy-17α-methylestra-5,15-dien-11β-yl][1,1'-biphenyl]-4-carbonitril als weißen Schaum.

¹H-NMR (CDCl₃): δ= 7,68-7,75 m (4H,Ar); 7,42-7,55 m (4H,Ar); 5,85 dbr (J=6 Hz,1H,H-16); 5,72 dd (J=6, 2.5 Hz,1H,H-15); 5,57 m (1H, H-6); 3,90-4,00 m (4H,Ketal); 3,55 ddbr (J=5, 7 Hz,1H,H-11); 3,20 s (3H,OMe); 1,20 s (3H,C-20); 0,70 s (3H,C-18)

### b) 4'-17β-Methoxy-17α-methyl-3-oxoestra-4,15-dien-11β-yl][1,1'-biphenyl]-4-carbonitril

Wie unter Beispiel 1r) beschrieben werden 260 mg 4'-[3,3-[1,2-Ethandiylbis(oxy)]-17β-methoxy-17α-methylestra-5,15-dien-11β-yl][1,1'-biphenyl]-4-carbonitril mit 0,7 ml 4 normaler wäßriger Salzsäure in 14 ml Aceton umgesetzt. Man erhält nach Säulenchromatographie an Kieselgel mit einem Gemisch aus Hexan/Ethylacetat 218 mg 4'-17β-Methoxy-17α-methyl-3-oxoestra-4,15-dien-11β-yl][1,1'-biphenyl]-4-carbonitril als weißen Schaum.

¹H-NMR (CDCl₃): δ= 7,70-7,75 m (4H,Ar); 7,50-7,55 m (4H,Ar); 5,90 sbr (1H,H-4); 5,87 dbr (J=6 Hz,1H,H-16); 5,75 dd (J=6, 2.5 Hz,1H,H-15); 3,50 ddbr (J=5, 7 Hz,1H,H-11); 3,20 s (3H,OMe); 1,18 s (3H,C-20); 0,77 s (3H,C-18)

[α]²⁰_{D}=129,6° (CHCl₃; c=0,50)

### Beispiel 15

### 4'-[17β-Hydroxy-3-oxo-17α-(1-propinyl)estra-4,15-dien-11β-yl][1,1'-biphenyl]-4-carbonitril

### a) 3,3-[1,2-Ethandiylbis(oxy)]-11β-(4-hydroxyphenyl)estr-5-en-17-on

Analog zu Beispiel 1o) werden 20 g 3,3-[1,2-Ethandiylbis(oxy)]-11β-(4-methoxyphenyl)-estr-5-en-17-on und 13,4 g Natriummethanthiolat in 350 ml Dimethylformamid umgesetzt. Man erhält 19 g 3,3-[1,2-Ethandiylbis(oxy)]-11β-(4-hydroxyphenyl)estr-5-en-17-on, die ohne Reinigung in die Folgestufe eingesetzt werden.

b) 11β-[4-[[Dimethyl-(1,1-dimethylethyl)silyl]oxy]phenyl]-3,3-[1,2-ethandiylbis(oxy)]estr-5-en-17-on

8,9 g 3,3-[1,2-Ethandiylbis(oxy)]-11β-(4-hydroxyphenyl)estr-5-en-17-on werden in 90 ml Dimethylformamid gelöst. Man addiert 4,63 g Imidazol und 4,93 g Dimethyl-(1,1-dimethylethyl)silylchlorid und rührt 4 Stunden bei Raumtemperatur nach. Anschließend wird die Reaktionslösung auf Eiswasser gegossen und mit Ethylacetat extrahiert. Man wäscht die organische Phase mit gesättigter wäßriger Natriumchloridlösung, filtriert und engt am Vakuum ein. Das erhaltene Rohprodukt wird durch Säulenchromatographie an Kieselgel mit einem Gemisch aus Hexan/Ethylacetat gereinigt. Man erhält 10,27 g 11β-[4-[[Dimethyl-(1,1-dimethylethyl)silyl]oxy]phenyl]-3,3-[1,2-ethandiylbis(oxy)]estr-5-en-17-on als weißen Schaum.

¹H-NMR (CDCl₃): δ= 7,09 d (J=8 Hz,2H,Ar); 6,63 d (J=8 Hz,2H,Ar); 5,46-5,48 m (1H,H-6); 3,80-3,90 m (4H,Ketal); 3,30 ddbr (J=5, 7 Hz,1H,H-11); 1,95 s (3H,Propin); 0,88 s (9H,t-Bu); 0,10 s (6H,SiMe₂)

### c) 11β-[4-[[Dimethyl-(1,1-dimethylethyl)silyl]oxy]phenyl]-3,3-[1,2-ethandiylbis(oxy)]-17-[(trimethylsilyl)oxy]estra-5,16-dien

Aus 6 g 11β-[4-[[Dimethyl-(1,1-dimethylethyl)silyl]oxy]phenyl]-3,3-[1,2-ethandiylbis(oxy)]estr-5-en-17-on, 4,02 ml Diisopropylamin, 18,13 ml einer 1,6 molaren Lösung von Butyllithium in Hexan und 5,07 ml Trimethylchlorsilan werden in 250 ml Tetrahydrofuran analog zu Beispiel 1e) nach Umkristallisation aus Acetonitril 6,5 g 11β-[4-[[Dimethyl-(1,1-dimethylethyl)silyl]oxy]phenyl]-3,3-[1,2-ethandiylbis(oxy)]-17-[(trimethylsilyl)oxy]-estra-5,16-dien dargestellt.

### d) 11β-[4-[[Dimethyl-(1,1-dimethylethyl)silyl]oxy]phenyl]-3,3-[1,2-ethandiylbis(oxy)]estra-5,15-dien-17-on

Wie unter Beispeil 1f) beschrieben werden aus 1,65 g 11β-[4-[[Dimethy1-(1,1-dimethylethyl)silyl]oxy]phenyl]-3,3-[1,2-ethandiylbis(oxy)]-17-[(trimethylsilyl)oxy]estra-5,16-dien und 747 mg Palladium(II)acetat in 60 ml Acetonitril nach Säulenchromatographie an Kieselgel mit einem Gemisch aus Hexan/Ethylacetat 1,23 g 11β-[4-[[Dimethyl-(1,1-dimethylethyl)silyl]oxy]phenyl]-3,3-[1,2-ethandiylbis(oxy)]estra-5,15-dien-17-on als weißer Schaum erhalten.

¹H-NMR (CDCl₃): δ= 7,52 dd (J=6, 1 Hz,1H,H-15); 7,19 d (J=8 Hz,2H,Ar); 6,73 d (J=7 Hz,2H,Ar); 5,98 dd (J=8, 2.5 Hz,1H,H-16); 5,50 m (1H,H-6); 3,90-4,00 m (4H,Ketal); 3,50 dd (J=5, 7 Hz,1H,H-11); 1,98 s (3H,Propin); 0,96 (9H, t-Bu); 0,90 s (3H,C-18); 0,20 s (6H,SiMe₂)

### e) 11β-[4-[[Dimethyl-(1,1-dimethylethyl)silyl]oxy]phenyl]3,3-[1,2-ethandiylbis(oxy)]-17α-(1-propinyl)estra-5,15-dien-17β-ol

Wie unter Beispiel 8a) beschrieben werden 3,5 g 11β-[4-[[Dimethyl-(1,1-dimethylethyl)silyl]oxy]phenyl]-3,3-[1,2-ethandiylbis(oxy)]estra-5,15-dien-17-on, 42 ml einer 1,6 molaren Lösung von Butyllithium und 350 ml mit Propin gesättigtes Tetrahydrofuran umgesetzt. Man erhält 3,7 g 11β-[4-[[Dimethyl-(1,1-dimethylethyl)silyl]oxy]phenyl]-3,3-[1,2-ethandiylbis(oxy)]-17α-(1-propinyl)estra-5,15-dien-17β-ol, welches ohne Reinigung in die Folgestufe eingesetzt wird.

### f) 3,3-[1,2-Ethandiylbis(oxy)]-11β-(3-(4-hydroxyphenyl)-17α-(1-propinyl)estra-5,15-dien-17β-ol

1,98 g 11β-[4-[[Dimethyl-(1,1 -dimethylethyl)silyl]oxy]phenyl]3,3-[1,2-ethandiylbis(oxy)]-17α-(1-propinyl)estra-5,15-dien-17β-ol werden in 35 ml Tetrahydrofuran gelöst. Man addiert 2,78 g Tetrabutylammoniumfluorid-trihydrat und rührt 1,5 Stunden bei Raumtemperatur nach. Anschließend wird die Reaktionslösung auf gesättigte Natriumhydrogencarbonatlösung gegossen und mit Ethylacetat extrahiert. Die organische Phase wird mit gesättigter wäßriger Natriumchloridlösung gewaschen, über Natriumsulfat getrocknet, filtriert und am Vakuum eingeengt. Man erhält nach Chromatographie an Kieselgel mit einem Gemisch aus Hexan/Ethylacetat 1,22 g 3,3-[1,2-Ethandiylbis(oxy)]-11β-(4-hydroxyphenyl)-17α-(1-propinyl)estra-5,15-dien-17β-ol als weißen Schaum.

¹H-NMR (CDCl₃): δ= 7,15 d (J=8 Hz,2H,Ar); 6,70 d (J=8 Hz,2H,Ar); 6,00 dbr (J=6 Hz,1H,H-16); 5,54 dd (J=6, 2.5 Hz,1H,H-15); 5,53 m (1H,H-6); 3,90-4,00 m (4H,Ketal); 3,50 ddbr (J=5, 7 Hz,1H-H-11); 3,40 s (1H,Phenol); 1,98 s (3H,Propin); 0,67 s (3H,C-18)

### g) 3,3-[1,2-Ethandiylbis(oxy)]-17α-(1-propinyl)-11β-[4-[[(1,1,2,2,3,3,4,4,4-nonafluorbutyl)-sulfonyl]oxy]phenyl]estra-5,15-dien-17β-ol

Analog zu Beispiel 10b) werden 1,22 g 3,3-[1,2-Ethandiylbis(oxy)]-11β-(4-hydroxyphenyl)-17α-(1-propin yl)estra-5,15-dien-17β-ol, 1,9 ml einer 1,6 molaren Lösung von n-Butyllithium und 1,09 ml Nonafluorbutansulfonylfluorid in 70 ml Tetrahydrofuran umgesetzt. Man erhält nach Säulenchromatographie an Kieselgel mit einem Gemisch aus Hexan/Ethylacetat 1,7 g 3,3-[1,2-Ethandiylbis(oxy)]-17α-(1-propinyl)-11β-[4-[[(1,1,2,2,3,3,4,4,4-nonafluorbutyl)-sulfonyl]oxy]phenyl]estra-5,15-dien-17β-ol als weißen Schaum.

¹H-NMR (CDCl₃): δ= 7,40 d (J=8 Hz,2H,Ar); 7,15 d (J=8 Hz,2H,Ar); 5,98 dbr (J=6 Hz,1H,H-16); 5,65 dd (J=6, 2.5 Hz,1H,H-15); 5,55 m (1H,H-6); 3,90-4,00 (4H,Ketal); 3,59 ddbr (J=5, 7 Hz,1H,H-11); 1,98 s (3H,Propin); 0,61 s (3H,C-18)

### h) 4'-[3,3-[1,2-Ethandiylbis(oxy)]-17β-hydroxy-17α-(1-propinyl)estra-5,15-dien-11β-yl]-[1,1'-biphenyl]-4-carbonitril

Analog zu Beispiel 2a) werden 560 mg 3,3-[1,2-Ethandiylbis(oxy)]-17α-(1-propinyl)-11β-[4-[[(1,1,2, 2, 3,3,4,4,4-nonafluorbutyl)sulfonyl]oxy]phenyl]estra-5,15-dien-17β-ol, 172 mg (4-Cyanphenyl)boronsäure, 45 mg Tetrakis(triphenylphosphin)palladium, 65 mg Lithiumchlorid und 0,96 ml einer 2 molaren wäßrigen Natriumcarbonatlösung in 7 ml Toluol und 3 ml Ethanol umgesetzt. Man erhält nach Säulenchromatographie an Kieselgel mit einem Gemisch aus Hexan/Ethylacetat 300 mg 4'-[3,3-[1,2-Ethandiylbis(oxy)]-17β-hydroxy-17α-(1-propinyl)estra-5,15-dien-11β-yl][1,1'-biphenyl]-4-carbonitril als weißen Schaum.

¹H-NMR (CDCl₃): δ= 7,70-7,75 m (4H,Ar); 7,47-7,55 m (4H,Ar); 6,02 dbr (J=6 Hz,1H,H-16); 5,69 dd (J=6, 2.5 Hz,1H,H-15); 3,90-4,00 m (4H,Ketal); 3,61 ddbr (J=5, 7 Hz,1H,H-11); 1,98s (3H,Propin); 0,70 s (3H,C-18)

### i) 4'-[17β-Hydroxy-3-oxo-17α-(1-propinyl)estra-4,15-dien-11β-yl][1,1'-biphenyl]4-carbonitril

Analog zu Beispiel 1r) werden 300 mg 4'-[3,3-[1,2-Ethandiylbis(oxy)]-17β-hydroxy-17α-(1-propinyl)estra-5,15-dien-11β-yl][1,1'-biphenyl]-4-carbonitril und 1,2 ml 4 normale wäßrige Salzsäure in 20 ml Aceton umgesetzt. Man erhält nach Säulenchromatographie an Kieselgel mit einem Gemisch aus Hexan/Ethylacetat 240 mg 4'-[17β-Hydroxy-3-oxo-17α-(1-propinyl)estra-4,15-dien-11β-yl][1,1'-biphenyl]-4-carbonitril als weißen Schaum.

¹H-NMR (CDCl₃): δ= 7,68-7,72 m (4H,Ar); 7,45-7,50 m (4H,Ar); 6,02 dbr (J=6 Hz,1H,H-16); 5,90 sbr (1H,H-4); 5,71 dd (J=6, 2.5 Hz,1H,H-15); 3,54 ddbr (J=5, 7 Hz,1H,H-11); 2,00 s (3H,Propin); 0,78 s (3H,C-18)

[α]²⁰_{D}=-89° (CHCl₃/Methanol;c=0,50)

### Beispiel 16

### 11β-[4-(3-Furanyl)phenyl]-17β-hydroxy-17α-(1-propinyl)estra-4,15-dien-3-on

### a) 3,3-[1,2-Ethandiylbis(oxy)]-11β-[4-(3-furanyl)phenyl]-17α-(1-propinyl)estra-5,15-dien-17β-ol

Analog zu Beispiel 1q werden aus 560 mg 3,3-[1,2-Ethandiylbis(oxy)]-17α-(1-propinyl)-11β-[4-[[(1,1,2,2, 3,3,4,4,4-nonafluorbutyl)sulfonyl]oxy]phenyl]estra-5,15-dien-17β-ol, 0,58 g (3-Furanyl)tributylstannan, 45 mg Tetrakis(triphenylphosphin)palladium, 65 mg Lithiumchlorid in 10 ml Dioxan nach Säulenchromatographie an Kieselgel mit einem Gemisch aus Hexan/Ethylacetat 286 mg 3,3-[1,2-Ethandiylbis(oxy)]-11β-[4-(3-furanyl)-phenyl]-17α-(1-propinyl)estra-5,15-dien-17β-ol als weißer Schaum erhalten.

¹H-NMR (CDCl₃): δ= 7,72 dbr (J=1.3 Hz,1H,Fu-2); 7,48 dd (J=1.8, 1.3 Hz,1H,Fu-5); 7,30-7,40 m (4H,Ar); 6,70 dbr (J=1.8 Hz,1H,Fu-4); 6,00 dbr (J=6 Hz,1H,H-16); 5,68 dd (J=6, 2.5 Hz,1H,H-15); 5,56 m (1H,H-6); 3,90-4,00 m (4H,Ketal); 3,56 ddbr (J=5, 7 Hz,1H,H-11); 1,91 s (3H,Propin); 0,70 s (3H,C-18)

### b) 11β-[4-(3-Furanyl)phenyl]-17β-hydroxy-17α-(1-propinyl)estra-4,15-dien-3-on

Analog zu Beispiel 1r) werden 286 mg 3,3-[1,2-Ethandiylbis(oxy)]-11β-[4-(3-furanyl)-phenyl]-17α-(1-propinyl)estra-5,15-dien-17β-ol und 0,45 ml 4 normale wäßrige Salzsäure in 10 ml Aceton umgesetzt. Man erhält nach Chromatographie an Kieselgel mit einem Gemisch aus Hexan/Ethylacetat 220 mg 11β-[4-(3-Furanyl)phenyl]-17β-hydroxy-17α-(1-propinyl)-estra-4,15-dien-3-on als weißen Schaum

¹H-NMR (CDCl₃): δ= 7,72 dbr (J=1.3 Hz,1H,Fu-2); 7,48 dd (J=1.8, 1.3 Hz,1H-Fu-5); 7,38-7,45 m (4H,Ar); 6,70 dbr (1.8 Hz,1H,Fu-4); 6,00 dbr (J=6 Hz,1H,H-15); 5,88 sbr (1H,H-4); 5,70 dd (J=6, 2.5 Hz,1H,H-15); 3,50 ddbr (J=5, 7 Hz,1H,H-11); 1,90 s (3H,Propin); 0,71 s (3H,C-18)

### Beispiel 17

### 11β-(4-Acetylphenyl)-17β-hydroxy-17α-(1-propinyl)estra-4,15-dien-3-on

### a) 3,3-[1,2-Ethandiylbis(oxy)]-11β-[4(1-ethoxyethenyl)phenyl]-17α-(1-propinyl)estra-5,15-dien-17β-ol

Analog zu Beispiel 1q) werden 560 mg 3,3-[1,2-Ethandiylbis(oxy)]-17α-(1-propinyl)-11β-[4-[[(1,1,2,2,3,3,4,4,4-nonafluorbutyl)sulfonyl]oxy]phenyl]estra-5,15-dien-17β-ol, 0,34 ml (1-Ethoxyvinyl)tributylzinn, 45 mg Tetrakis(triphenylphosphin)palladium, 65 mg Lithiumchlorid in 10 ml Dioxan umgesetzt. Man erhält nach Säulenchromatographie an Kieselgel mit einem Gemisch aus Hexan/Ethylacetat 277 mg 3,3-[1,2-Ethandiylbis(oxy)]-11β-[4-(1-ethoxyethenyl)phenyl]-17α-(1-propinyl)estra-5,15-dien-17β-ol als weißen Schaum.

### b) 11β-(4-Acetylphenyl)-17β-hydroxy-17α-(1-propinyl)estra-4,15-dien-3-on

Analog zu Beispiel 1r) werden 277 mg 3,3-[1,2-Ethandiylbis(oxy)]-11β-[4-(1-ethoxy-ethenyl)phenyl]-17α-(1-propinyl)estra-5,15-dien-17β-ol und 0,43 ml 4 normale wäßrige Salzsäure in 10 ml Aceton umgesetzt. Man erhält nach Säulenchromatographie an Kieselgel mit einem Gemisch aus Hexan/Ethylacetat 200 mg 11β-(4-Acetylphenyl)-17β-hydroxy-17α-(1-propinyl)estra-4,15-dien-3-on als weißen Schaum.

¹H-NMR (CDCl₃): δ= 7,90 d (J=8 Hz,2H,Ar); 7,52 d (J=8 Hz,2H,Ar); 6,00 dbr (J=6 Hz,1H,H-16); 5,90 sbr (1H,H-4); 5,70 dd (J=6, 2.5 Hz,1H,H-15); 3,54 ddbr (J=5, 7 Hz,1H,H-11); 1,95 s (3H,Propin); 0,72 s (3H,C-18)

### Beispiel 18

### 4'-[4',5'-Dihydro-3-oxospiro[estra-4,15-dien-17β,2'(3'H)-furan]-11β-yl][1,1'-biphenyl]-4-carbonitril

### a) 3,3-[1,2-Ethandiylbis(oxy)]-11β-(4-methoxyphenyl)-17α-(2-propenyl)estra-5,15-dien-17β-ol

Zu 12 ml einer 2 molaren Lösung von Allylmagnesiumchlorid in Tetrahydrofuran werden bei 0°C unter Argon 2 g 3,3-[1,2-Ethandiylbis(oxy)]-11β-(4-methoxyphenyl)estra-5,15-dien-17-on, gelöst in 60 ml absolutem Tetrahydrofuran getropft. Man läßt 30 Minuten bei 0°C nachrühren und quencht dann mit gesättigter Ammoniumchloridlösung. Man extrahiert mit Ethylacetat, wäscht die organische Phase mit gesättigter wäßriger Natriumchloridlösung, trocknet über Natriumsulfat, filtriert und engt am Vakuum ein. Nach Säulenchromatographie an Kieselgel mit einem Gemisch aus Hexan/Ethylacetat werden 1,58 g 3,3-[1,2-Ethandiylbis(oxy)]-11β-(4-methoxyphenyl)-17α-(2-propenyl)estra-5,15-dien-17β-ol als weißer Schaum erhalten.

¹H-NMR (CDCl₃): δ= 7,23 d (J=8 Hz,2H,Ar); 6,78 d (J=8 Hz,2H,Ar); 5,80-5,95 m (2H); 5,55 m (2H); 5,10-5,15 m (2H); 3,90-4,00 m (4H,Ketal); 3,80 s (3H,OMe); 3,48 ddbr (J=5, 7 Hz,1H,H-11); 0,70 s (3H,C-18)

### b) 3,3-[1,2-Ethandiylbis(oxy)]-17α-(3-hydroxypropyl)11β-(4-methoxyphenyl)estra-5,15-dien-17β-ol

Zu 463 mg 3,3-[1,2-Ethandiylbis(oxy)]-11β-(4-methoxyphenyl)-17α-(2-propenyl)estra-5,15-dien-17β-ol in 6 ml absolutem Tetrahydrofuran werden unter Argon 6 ml einer 0,5 molaren Lösung von 9-Borabicyclo[3.3.1]nonan in Tetrahydrofuran gegeben. Man läßt 12 Stunden bei Raumtemperatur nachrühren, addiert dann 5,3 ml einer 2,5 normalen wäßrigen Natriumhydroxidlösung sowie 3,2 ml 30%iges Wasserstoffperoxid und kocht 1 Stunde unter Rückfluß. Anschließend läßt man abkühlen und extrahiert mit Ethylacetat Die organische Phase wird mit Wasser, gesättigter Natriumthiosulfatlösung sowie mit gesättigter Natriumchloridlösung gewaschen, über Natriumsulfat getrocknet, filtriet und am Vakuum eingeengt. Man erhält nach Säulenchromatographie an Kieselgel mit einem Gemisch aus Hexan/Ethylacetat 450 mg 3,3-[1,2-Ethandiylbis(oxy)]-17α-(3-hydroxypropyl)-11β-(4-methoxyphenyl)estra-5,15-dien-17β-ol.

¹H-NMR (CDCl₃): δ= 7,20 d (J=8 Hz,2H,Ar); 6,79 d (J=8 Hz,2H,Ar); 5,92 dbr (J=7 Hz,1H,H-16); 5,67 dd (J=7, 2.5 Hz,1H,H-15); 5,55 m (1H,H-6); 3,90-4,00 m (4H,Ketal); 3,65-3,70 m (2H,CH₂OH); 3,47 ddbr (J=5,7 Hz,1H,H-11); 0,68 s (3H,C-18)

### c) 4-[4',5'-Dihydro-3,3-[1,2-ethandiylbis(oxy)]spiro[estra-5,15-dien-17β,2'(3'H)-furan]11β-yl]-phenol

Analog zu Beispiel 1o) werden aus 2,24 g 3,3-[1,2-Ethandiylbis(oxy)]-17α-(3-hydroxy-propyl)-11β-(4-methoxy phenyl)estra-5,15-dien-17β-ol und 1,34 g Natriummethamthiolat in 30 ml Dimethylformamid nach Säulenchromatographie an Kieselgel mit einem Gemisch aus Hexan/Ethylacetat 1,25 g 4-[4',5'-Dihydro-3,3-[1,2-ethandiylbis(oxy)]spiro[estra-5,15-dien-17β,2'(3'H)-furan]- 11β-yl]phenol erhalten.

¹H-NMR (CDCl₃): δ= 7,15 d (J=8 Hz,2H,Ar); 6,70 d (J=8 Hz,2H,Ar); 5,83 dbr (J=7 Hz,1H,H-16); 5,65 dd (J=7, 2.5 Hz,1H,H-15); 5,55 m (1H,H-6); 5,40 s (1H,Phenol); 3,90-4,00 m (4H,Ketal); 3,75-3,83 m (2H,CH₂OH); 3,43 ddbr (J=5, 7 Hz,1H,H-11); 0,71 s (3H,C-18)

### d) 4',5'-Dihydro-3,3-[1,2-ethandiylbis(oxy)]-11β-[4-[[(1,1,2,2,3,3,4,4,4-nonafluorbutyl)-sulfonyl]oxy]phenyl]spiro[estra-5,15-dien-17β,2'(3'H)-furan]

Analog zu Beispiel 10b) werden 1,25 g 4-[4',5'-Dihydro-3,3-[1,2-ethandiylbis(oxy)]-spiro[estra-5,15-dien-17β,2'(3'H)-furan]-11β-yl]phenol, 1,9 ml einer 1,6 molaren Lösung von Butyllithium in Hexan und 0,84 ml Nonafluorbutansulfonylfluorid in 70 ml absolutem Tetrahydrofuran umgesetzt Man erhält 1,9 g 4',5'-Dihydro-3,3-[1,2-ethandiylbis(oxy)]-11β-[4-[[(1,1,2,2,3,3,4,4,4-nonafluorbutyl)sulfonyl]oxy]phenyl]spiro[estra-5,15-dien-17β,2'(3'H)-furan], welches ohne Reinigung in die Folgestufe eingesetzt wird.

### e) 4'-[4',5'-Dihydro-3,3-[1,2-ethandiylbis(oxy)]spiro[estra-5,15-dien-17β,2'(3'H)-furan]-11β-yl][1,1'-biphenyl]-4-carbonitril

Analog zu Beispiel 2a) werden aus 2,04 g 4',5'-Dihydro-3,3-[1,2-ethandiylbis(oxy)]-11β-[4-[[(1,1,2,2,3,3,4,4,4-nonafluorbutyl)sulfonyl]oxy]phenyl]spiro[estra-5,15-dien-17β,2'(3'H)-furan], 590 mg (4-Cyanphenyl)boronsäure, 161 mg Tetrakis(triphenylphosphin)palladium, 237 mg Lithiumchlorid und 3,5 ml einer 2 molaren wäßrigen Natriumcarbonatlösung in 21 ml Toluol und 9 ml Ethanol nach Säulenchromatographie an Kieselgel mit einem Gemisch aus Hexan/Ethylacetat 1,16 g 4'-[4',5'-Dihydro-3,3-[1,2-ethandiylbis(oxy)]spiro[estra-5,15-dien-17β,2'(3'H)-furan]-11β-yl][1,1'-biphenyl]-4-carbonitril als weißer Schaum erhalten.

¹H-NMR (CDCl₃): δ= 7,70-7,75 m (4H,Ar); 7,45-7,55 m (4H,Ar); 5,87 dbr (J=7 Hz,1H,H-16); 5,67 dd (J=7, 2.5 Hz,1H,H-15); 5,50 m (1H,H-6); 3,90-4,00 m (4H,Ketal); 3,73-3,80 m (2H,CH₂O); 3,54 ddbr (J=5, 7 Hz,1H,H-11); 0,73 s (3H,C-18)

### f) 4'-[4',5'-Dihydro-3-oxospiro[estra-4,15-dien-17β,2'(3'H)-furan]-11β-yl][1,1'-biphenyl]-4-carbonitril

Analog zu Beispiel 1r) werden 1,16 g 4'-[4',5'-Dihydro-3,3-[1,2-ethandiylbis(oxy)]-spiro[estra-5,15-dien-17β,2'(3'H)-furan]-11β-yl][1,1'-biphenyl]-4-carbonitril, 2,9 ml 4 normale wäßrige Salzsäure in 60 ml Aceton umgesetzt Man erhält nach Säulenchromatographie an Kieselgel mit einem Gemisch aus Hexan/Ethylacetat 850 mg 4'-[4',5'-Dihydro-3-oxospiro[estra-4,15-dien-17β,2'(3'H)-furan]-11β-yl][1,1'-biphenyl]-4-carbonitril als weißen Schaum.

¹H-NMR (CDCl₃): δ= 7,68-7,75 m (4H,Ar); 7,50-7,53 m (4H,Ar); 5,90 sbr (1H,H-4); 5,89 dbr (J=7 Hz,1H,H-16); 5,70 dd (J=7, 2.5 Hz,1H,H-15); 3,73-3,80 m (2H,CH₂O); 3,50 ddbr (J=5, 7 Hz,1H,H-11); 0,80 s (3H,C-18)

[α]²⁰_{D}=+87,8° (CHCl₃; c=0,510)

### Beispiel 19

### 4'-[17α-Ethinyl-17β-hydroxy-3-oxoestra-4,15-dien-11β-yl][1,1'-biphenyl]-4-carbonitril

### a) 11β-[4-[[Dimethyl-(1,1-dimethylethyl)silyl]oxy]phenyl]-3,3-[1,2-ethandiylbis(oxy)]-17α-ethinylestra-5,15-dien-17β-ol

Wie unter Beispiel 8a) beschrieben werden 2,5 g 11β-[4[[Dimethyl-(1,1-dimethylethyl)-silyl]oxy]phenyl]-3,3-[1,2 -ethandiylbis(oxy)]estra-5,15-dien-17-on, 30 ml einer 1,6 molaren Lösung von n-Butyllithium in Hexan sowie 250 ml mit Ethin gesättigtes Tetrahydrofuran umgesetzt. Man erhält nach Säulenchromatographie an Kieselgel mit einem Gemisch aus Hexan/Ethylacetat 2,1 g 11β-[4-[[Dimethyl-(1,1-dimethylethyl)silyl]oxy]phenyl]-3,3-[1,2-ethandiylbis(oxy)]-17α-ethinylestra-5,15-dien-17β-ol als weißen Schaum.

¹H-NMR (CDCl₃): δ= 7,17 d (J=8 Hz,2H,Ar); 6,72 d (J=8 Hz,2H,Ar); 6,04 dbr (J=6 Hz,1H,H-16); 5,69 dd (J=6, 2.5 Hz,1H,H-15); 5,53 m (1H,H-6); 3,90-4,00 m (4H,Ketal); 3,50 ddbr (J=5, 7 Hz,1H,H-11); 2,62 s (1H,Ethin); 0,98 s (9H,t-Bu); 0,70 s (3H,C-18); 0,20 s (6H,SiMe₂)

### b) 3,3-[1,2-Ethandiylbis(oxy)]-17α-ethinyl-11β-(4-hydroxyphenyl)estra-5,15-dien-17β-ol

Wie unter Beispiel 15f) beschrieben werden 2,1 g 11β-[4-[[Dimethyl-(1,1-dimethylethyl)-silyl]oxy]phenyl]-3,3-[1,2-ethandiylbis(oxy)]-17α-ethinylestra-5,15-dien-17β-ol und 3,1 g Tetrabutylammoniumfluorid-trihydrat in 60 ml absolutem Tetrahydrofuran umgesetzt. Man erhält 1,6 g 3,3-[1,2-Ethandiylbis(oxy)]-17α-ethinyl-11β-(4-hydroxyphenyl)estra-5,15-dien-17β-ol, welches ohne Aufreinigung in die Folgestufe eingesetzt wird.

### c) 3,3-[1,2-Ethandiylbis(oxy)]-17α-ethinyl-11β-[4-[[(1,1,2,2,3,3,4,4,4-nonafluorbutyl)sulfonyl]-oxy]phenyl]estra-5,15-dien-17β-ol

1,46 g 3,3-[1,2-Ethandiylbis(oxy)]-17α-ethinyl-11β-(4-hydroxyphenyl)estra5,15-dien-17β-ol, 1,01 ml Nonafluorbutansulfonylfluorid und 2,32 ml einer 1,6 molaren Lösung von Butyllithium in Hexan werden in 80 ml absolutem Tetrahydrofuranwie unter Beispiel 10b) beschrieben umgesetzt. Man erhält 2,33 g 3,3-[1,2-Ethandiylbis(oxy)]-17α-ethinyl-11β-[4-[[(1,1,2,2,3,3,4,4,4-nonafluorbutyl)sulfonyl]oxy]phenyl]estra-5,15-dien-17β-ol, welches ohne Reinigung in die Folgestufe eingesetzt wird

### d) 4'-[3,3-[1,2-Ethandiylbis(oxy)]-17α-ethinyl-17β-hydroxyestra-5,15-dien-11β-yl][1,1'-biphenyl]-4-carbonitril

1,3 g 3,3-[1,2-Ethandiylbis(oxy)]-17α-ethinyl-11β-[4[[(1,1,2,2,3,3,4,4,4-nonafluorbutyl)sulfonyl]oxy]phenyl]estra-5,15-dien-17β-ol, 384 mg (4-Cyanphenyl)boronsäure, 105 mg Tetrakis(triphenylphosphin)palladium, 155 mg Lithiumchlorid und 2,3 ml einer 2 molaren wäßrigen Natriumcarbonatlösung werden in 15 ml Toluol und 6 ml Ethanol analog zu Beispiel 2a) umgesetzt. Man erhält nach Säulenchromatographie an Kieselgel mit einem Gemisch aus Hexan/Ethylacetat 540 mg 4'-[3,3-[1,2-Ethandiylbis(oxy)]-17α-ethinyl-17β-hydroxyestra-5,15-dien-11β-yl][1,1'-biphenyl]-4-carbonitril als weißen Schaum.

¹H-NMR (CDCl₃): δ= 7,70-7,75 m (4H,Ar); 7,45-7,50 m (4H,Ar); 6,05 dbr (J=6 Hz,1H,H-16); 5,70 dd (J=6, 2.5 Hz,1H,H-15); 5,58 m (1H-H-6); 3,90-4,00 m (4H-Ketal); 3,62 ddbr (J=5, 7 Hz,1H,H-11); 2,66 s (1H,Ethin); 0,72 s (3H,C-18)

### e) 4'-[17α-Ethinyl-17β-hydroxy-3-oxoestra-4,15-dien-11β-yl][1,1'-biphenyl]-4-carbonitril

Analog zu Beispiel 1r) 540 mg 4'-[3,3-[1,2-Ethandiylbis(oxy)]-17α-ethinyl-17β-hydroxy-estra-5,15-dien-11β-yl][1,1'-biphenyl]-4-carbonitril und 1,35 ml 4 normale wäßrige Salzsäure in 30 ml Aceton umgesetzt. Man erhält nach Säulenchromatographie an Kieselgel mit einem Gemisch aus Hexan/Ethylacetat 330 mg 4'-[17α-Ethinyl-17β-hydroxy-3-oxoestra-4,15-dien-11β-yl][1,1'-biphenyl]-4-carbonitril als weißen Schaum.

¹H-NMR (CDCl₃): δ= 7,70-7,75 m (4H,Ar); 7,50-7,55 m (4H,Ar); 6,07 dbr (J=6 Hz,1H,H-16); 5,90 sbr (1H,H-4); 5,73 dd (J=6, 2.5 Hz,1H,H-15); 3,55 ddbr (J=5, 7 Hz,1H,H-11); 2,70s (1H,Ethin); 0,80 s (3H,C-18)

**TABELLE 1**

| Testverbindung | Dosis mg/Tier/Tag p.o. | Abortrate n Abort/ n gesamt | % |
|---|---|---|---|
| B | 1,0 | 4/4 | 100 |
| | 0,3 | 4/4 | 100 |
| | 0,1 | 4/4 | 100 |
| | 0,03 | 1/4 | 25 |
| C | 1,0 | 4/4 | 100 |
| | 0,3 | 4/4 | 100 |
| E | 1,0 | 4/4 | 100 |
| | 0,3 | 4/4 | 100 |
| | 0,1 | 4/4 | 100 |
| | 0,03 | 2/4 | 50 |
| F | 1,0 | 4/4 | 100 |
| | 0,3 | 4/4 | 100 |
| | 0,1 | 4/4 | 100 |
| | 0,03 | 4/4 | 100 |

## Patentansprüche

1. Verbindungen der allgemeinen Formel I 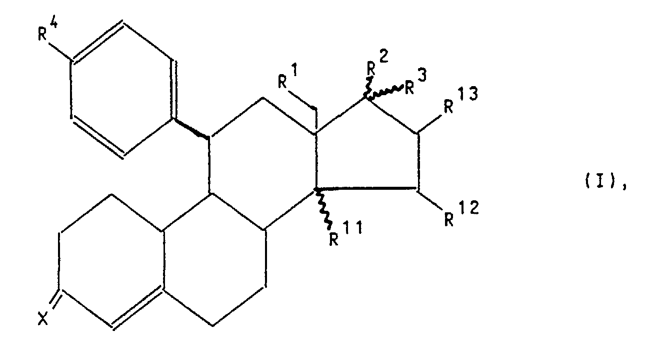 worin entweder
Ia) R¹¹ ein β-ständiges Wasserstoffatom sowie R¹² und R¹³ je ein Wasserstoffatom oder
Ib) R¹¹ ein β-ständiges Wasserstoffatom sowie R¹² und R¹³ gemeinsam eine zweite Bindung oder
Ic) R¹¹ und R¹² gemeinsam eine zweite Bindung sowie R¹³ ein Wasserstoffatom oder
Id) R¹¹ ein α-ständiges Wasserstoffatom sowie R¹² und R¹³ gemeinsam eine zweite Bindung
bedeuten sowie in Ia), Ib), Ic) oder Id)
X für ein Sauerstoffatom, die Hydroxyiminogruppierung >N~OH oder zwei Was serstoffatome,
R¹ für ein Wasserstoffatom oder eine Methylgruppe,
R² für eine Hydroxygruppe, eine C₁-C₁₀-Alkoxy- oder C₁-C₁₀-Acyloxygruppe,
R³ für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest -OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor-Brom-oder Jod-Atom, einen C₁-C₁₀-Hydroxyalkyl-, C₁-C₁₀-Alkoxyalkyl-, C₁-C₁₀-Acyloxyalkylrest oder wenn m = 0 ist, eine Methylgruppe bedeuten, ferner die Gruppierung -(CH₂)ₚ-CH=CH-(CH₂)ₖCH₂R⁶, wobei p 0 oder 1 und k 0, 1 oder 2 und R⁶ ein Wasserstoffatom, eine Hydroxygruppe, einen C₁-C₄-Alkoxy- oder C₁-C₄-Acyloxyrest bedeuten,
wobei
in Ia) und Ib) R² α- und R³ β-ständig und
in Ic) und Id) R² β- und R³ α-ständig ist,
oder aber R² und R³ gemeinsam für einen Rest der Formel 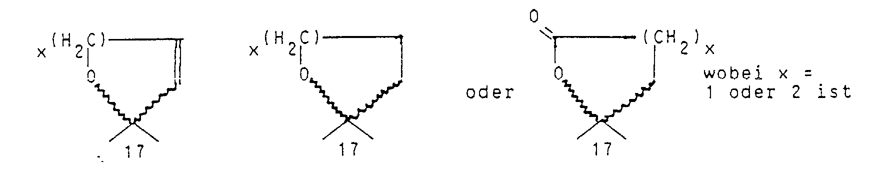 R⁴ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten C₁-C₈-Alkyl-, -Acyl-oder Alkoxyalkylrest, für
eine 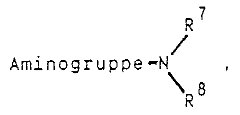 in welcher R⁷ und R⁸ unabhängig voneinander ein Wasserstoffatom oder eine C₁-C₄-Alkylgruppe bedeuten, oder für ein entsprechendes Aminoxid 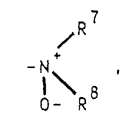 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα 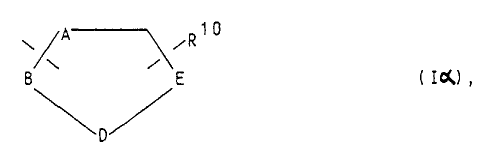 in welchem A ein Stickstoff-, Sauerstoff- oder Schwefelatom, -B-D-E- die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl-oder Alkoxyalkylrest, für
eine 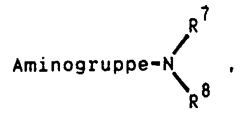 in welcher R⁷ und R⁸ unabhängig voneinander ein Wasserstoffatom oder eine C₁-C₄-Alkylgruppe bedeuten, oder ein entsprechendes Aminoxid 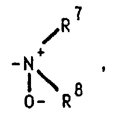 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ 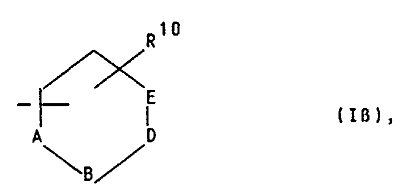 in welchem A ein Stickstoffatom und -B-D-E- die Elementenfolge -C-C-C-, -N-C-C-, -C-N-C- oder -C-C-N- bedeuten und R¹⁰ die bereits angegebene Bedeutung hat,
oder für einen Phenylrest der Formel Iγ 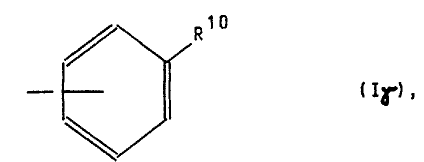 worin R¹⁰ die bereits angegebene Bedeutung hat,
stehen,
sowie deren pharmakologisch verträgliche Additionssalze mit Säuren.

2. Verbindungen nach Anspruch 1, nämlich
17α-Hydroxy-17β-(3-hydroxypropyl)-11β-[4-(3-pyridinyl)phenyl]-14β-estr-4-en-3-on
11-β-(4-acetylphenyl)-17α-hydroxy-17β-(3-hydroxypropyl)-14β-estr-en-3-on
11β-[4-(3-Furanyl)phenyl]-17α-hydroxy-17β-(3-hydroxypropyl]-14β-estr-4-en-3-on
4'-[17α-Hydroxy-17β-(3-hydroxypropyl)-3-oxo-14β-estr-4-en-11β-yl][1,1'-biphenyl]--4-carbonitril
(Z)-11β-(4-Acetylphenyl)-17α-hydroxy-17β-(3-hydroxy-1-propenyl)-14β-estr-en-3-on
(Z)-4'-[17α-Hydroxy-17β-(3-hydroxy-1-propenyl)-3-oxo-14β-estr-4-en-11β-yl][1,1'-biphenyl]-4-carbonitril
11β-(4-Acetylphenyl)-17α-hydroxy-17β-(methoxymethyl)-14β-estr-4-en-3-on
11β-(4-Acetylphenyl)-17α-hydroxy-3-oxo-14β-estr-4-en-17β-acetonitril
11β-[4-(3-Furanyl)phenyl]-17α-hydroxy-17β-(3-hydroxypropyl)-14β-estra-4,15-dien-3-on
4'-[17α-Hydroxy-17β-(3-hydroxypropyl)-3-oxo-14β-estra-4,15-dien-11β-yl)[1,1'-biphenyl]-4-carbonitril
11β-(4-Acetylphenyl)-17α-hydroxy-17β-methyl-14β-estra-4,15-dien-3-on
17β-Hydroxy-11β-(4-methoxyphenyl)-17α-methylestra-5,14-dien-3-on
11β-[4-(3-Furanyl)phenyl]-17β-hydroxy-17α-methylestra-5,14-dien-3-on
11β-(4-Acetylphenyl)-17β-hydroxy-17α-methylestra-5,14-dien-3-on
11β-(4-Acetylphenyl)-17β-hydroxy-17α-(3-hydroxypropyl)estra-5,14-dien-3-on
4'-[17β-Hydroxy-17α-(3-hydroxypropyl)-3-oxoestra-4,14-dien-11β-yl][1,1'-biphenyl]--4-carbonitril
11β-(4-Acetylphenyl)-17β-hydroxy-17α-(1-propinyl)estra-4,14-dien-3-on
11β-(4-Acetylphenyl)-4',5'-dihydrospiro[estra-4,14-dien-17β,2'(3'H)-furan]-3-on
11β-[4-(3-Furanyl)phenyl]-17β-hydroxy-17α-(3-hydroxypropyl)estra-4,15-en-3-on
4'-[17β-Hydroxy-17α-(3-hydroxypropyl)-3-oxoestra-4,15-dien-11β-yl][1,1'-biphenyl]-4-carbonitril
11β-[4-(3-Furanyl)phenyl]-17β-hydroxy-17α-methylestra-4,15-dien-3-on
11β-(4-Acetylphenyl)-17β-hydroxy-3-oxoestra-4,15-dien-17α-acetonitril
17-Hydroxy-17α-methyl-11β-[4-(3-thienyl)phenyl]estra-4,14-dien-3-on
17β-Hydroxy-11β-(4-methoxyphenyl)-17α-methylestra-4,15-dien-3-on
11β-(4-Acetylphenyl)-17β-hydroxy-17α-methylestra-4,15-dien-3-on
17β-Hydroxy-17α-methyl-11β-[4-(3-pyridinyl)phenyl]estra-4,15-dien-3-on
4'-[17β-Hydroxy-17α-methyl-3-oxoestra-4,15-dien-11β-yl][1,1'-biphenyl]-4-carbonitril
4'-[17β-Methoxy-17α-methyl-3-oxoestra-4,15-dien-11β-yl][1,1'-biphenyl]-4-carbonitril
17β-Hydroxy-11β-(4-methoxyphenyl)-17α-(1-propinyl)estra-4,15-dien-3-on
4'-[17β-Hydroxy-17α-(1-propinyl)-3-oxoestra-4,15-dien-11β-yl][1,1'-biphenyl]-4-carbonitril
11β-[4-(3-Furanyl)phenyl]-17β-hydroxy-17α-(1-propinyl)estra-4,15-dien-3-on
11β-(4-Acetylphenyl)-17β-hydroxy-17α-(1-propinyl)estra-4,15-dien-3-on
4'[4',5'-Dihydro-3-oxospiro[estra-4,15-dien-17β,2'(3'H)-furan]-11β-yl][1,1'-biphenyl]-4-carbonitril
(Z)-17β-Hydroxy-17α-3-hydroxy-1-propenyl)-11β-(4-methoxyphenyl)-estra-4,15-dien-3-on
11β-(4-Acetylphenyl)-17α-ethinyl-17β-hydroxyestra-4,15-dien-3-on
4'-[17α-Ethinyl-17β-Hydroxy-3-oxoestra-4,15-dien-11β-yl][1,1'-biphenyl]-4-carbonitril
11β-[4-(Dimethylamino)phenyl]-17β-hydroxy-17α-methylestra-4,15-dien-3-on

3. Verfahren zur Herstellung von Verbindungen der allgemeinen Formel I 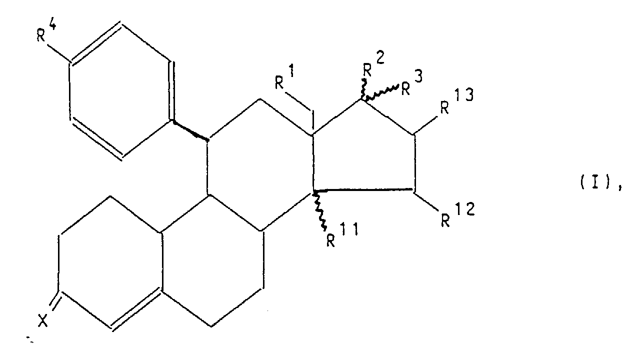 worin X, R¹, R², R³, R⁴, R¹¹, R¹² und R¹³ die in Anspruch 1 angegebene Bedeutung haben, dadurch gekennzeichnet, daß eine Verbindung der allgemeinen Formel II 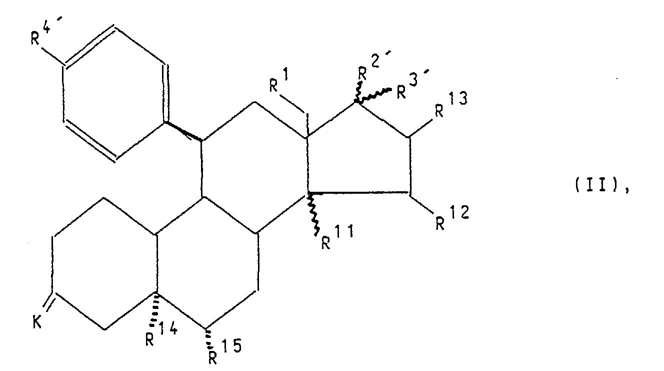 worin K eine als Ketal geschützte Ketogruppe ist, R¹, R¹¹, R¹² und R¹³ die gleiche Bedeutung wie in Formel I sowie R^{2'}, R^{3'} und R^{4'} unter Ausschluß des Cyanidrestes für R⁴ die gleiche Bedeutung wie R², R³ bzw. R⁴ in Formel I haben, wobei vorhandene Hydroxy-, Mercapto-, Amino-, Oxo- und/oder terminale Acetylengruppen gegebenenfalls geschützt sind, sowie R¹⁴ eine Hydroxygruppe und R¹⁵ ein Wasserstoff oder R¹⁴ und R¹⁵ gemeinsam eine Doppelbindung bedeuten, in bekannter Weise der Einwirkung eines sauren Agens zur Freisetzung der 3-Oxogruppe sowie der anderen geschützten Gruppen und gegebenenfalls zur Wasserabspaltung unterworfen und anschließend gewünschtenfalls in R² und/oder R³ und/oder R⁴ vorhandene Hydroxy-, Mercapto-und/oder Aminogruppe(n) alkyliert bzw. acyliert, gewünschtenfalls ein Cyanidrest in den 11β-Arylsubstituenten eingeführt, gewünschtenfalls die in R⁴ gegebenenfalls enthaltene Amino- oder Sulfidgruppe oxidiert, gewünschtenfalls mit Hydroxylamin-hydrochlorid zum Produkt der allgemeinen Formel I mit X in der Bedeutung der Hydroxyiminogruppierung 〉N~OH umgesetzt oder die 3-Oxogruppe in ein Produkt der allgemeinen Formel I, worin X für 2 Wasserstoffatome steht, umgewandelt sowie gegebenenfalls ein pharmazeutisch verträgliches Säureadditionssalz hergestellt wird/werden.

4. Pharmazeutische Präparate, die mindestens eine Verbindung gemäß Anspruch 1 oder 2 sowie einen pharmazeutischen Träger enthalten.

5. Verwendung der Verbindungen gemäß Anspruch 1 oder 2 zur Herstellung von Arzneimitteln.

## Claims

1. Compounds of the general formula I 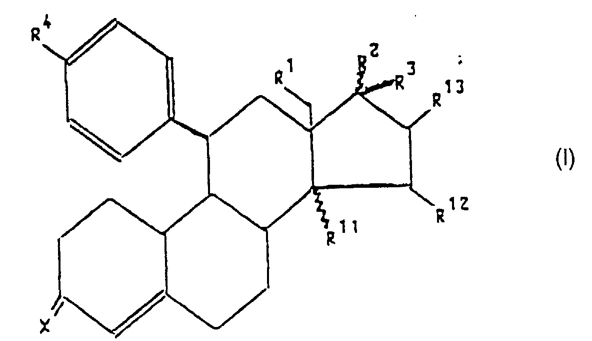 wherein
la) R¹¹ represents a hydrogen atom in the β configuration and each of R¹² and R¹³ represents a hydrogen atom, or
Ib) R¹¹ represents a hydrogen atom in the β configuration and R¹² and R¹³ together form a second bond, or
Ic) R¹¹ and R¹² together form a second bond and R¹³ represents a hydrogen atom, or
Id) R¹¹ represents a hydrogen atom in the α configuration and R¹² and R¹³ together form a second bond,
and in Ia), Ib), Ic) and Id)
X represents an oxygen atom, the hydroxyimino grouping > N∼OH or two hydrogen atoms,
R¹ represents a hydrogen atom or a methyl group,
R² represents a hydroxy group, or a C₁-C₁₀ alkoxy or C₁-C₁₀acyloxy group,
R³ represents a hydrogen atom; the grouping -(CH₂)ₙCH₂Z wherein n is 0, 1, 2, 3, 4 or 5 and Z is a hydrogen atom, the cyano group or the radical -OR⁵ wherein R⁵ = H, C₁-C₁₀alkyl or C₁-C₁₀acyl; the grouping -(CH₂)ₘC≡C-Y wherein m is 0, 1 or 2 and Y is a hydrogen, fluorine, chlorine, bromine or iodine atom, a C₁-C₁₀hydroxyalkyl, C₁-C₁₀alkoxyalkyl or C₁-C₁₀acyloxyalkyl radical, or, when m=0, a methyl group; or the grouping -(CH₂)ₚ-CH=CH-(CH₂)ₖCH₂R⁶ wherein p is 0 or 1, k is 0, 1 or 2 and R⁶ is a hydrogen atom, a hydroxy group or a C₁-C₄alkoxy or C₁-C₄acyloxy radical; wherein
in Ia) and Ib) R² is in the α configuration and R³ is in the β configuration, and
in Ic) and Id) R² is in the β configuration and R³ is in the α configuration, or altematively R² and R³ together form a radical of the formula 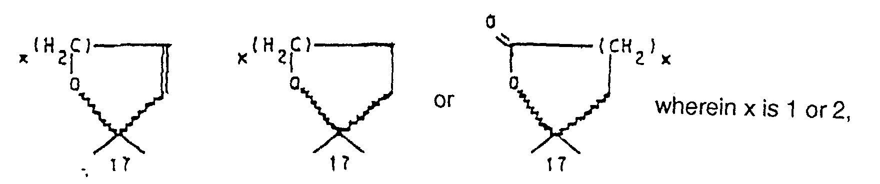
R⁴ is a hydrogen atom, a cyano group, a chlorine, fluorine, bromine or iodine atom, a trialkylsilyl or trialkylstannyl group, a straight-chain or branched, saturated or unsaturated C₁-C₈-alkyl, -acyl or -alkoxyalkyl radical;
an amino group 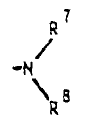 wherein R⁷ and R⁸ each independently of the other represents a hydrogen atom or a C₁-C₄alkyl group; or a corresponding amine oxide 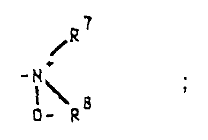 or the grouping -OR⁹ or -S(O)ᵢR⁹ wherein i = 0, 1 or 2, in which R⁹ is a hydrogen atom, methyl, ethyl, propyl, isopropyl, methoxyphenyl, ally or or 2-dimethylaminoethyl group; or a heteroaryl radical of the formula Iα 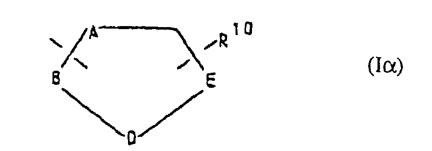 in which A is a nitrogen, oxygen or sulphur atom, -B-D-E- is the sequence of elements -C-C-C-, -N-C-C- or -C-N-C- and R¹⁰ is a hydrogen atom, a cyano group, a
chlorine, fluorine, bromine or iodine atom, a trialkylsilyl or trialkylstannyl group, a straight-chain or branched, saturated or unsaturated C₁-C₈-alkyl, -acyl or -alkoxyalkyl radical,
an amino group 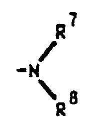 wherein R⁷ and R⁸ each independently of the other represents a hydrogen atom or a C₁-C₄alkyl group, or a corresponding amine oxide 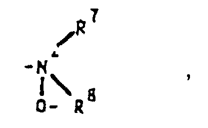 or the grouping -OR⁹ or -S(O)ᵢR⁹ wherein i = 0, 1 or 2, in which R⁹ is a hydrogen atom, a methyl, ethyl, propyl, isopropyl, methoxyphenyl, allyl or 2-dimethylaminoethyl group;
or a heteroaryl radical of the formula Iβ 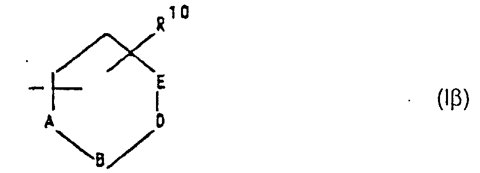 in which A is a nitrogen atom and -B-D-E- is the sequence of elements -C-C-C-, -N-C-C-, -C-N-C- or -C-C-N- and R¹⁰ has the meaning given above;
or a phenyl radical of the formula Iγ 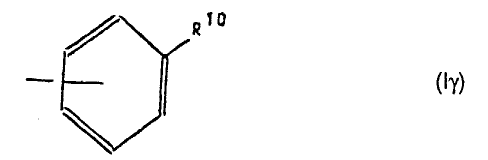 wherein R¹⁰ has the meaning given above;
and also their pharmacologically tolerable addition salts with acids.

2. Compounds according to claim 1, namely
17α-hydroxy-17β-(3-hydroxypropyl)-11β-[4-(3-pyridinyl)phenyl]-14β-oestr-4-en-3-one
11β-(4-acetylphenyl)-17α-hydroxy-17β-(3-hydroxypropyl)-14β-oestr-4-en-3-one
11β-[4-(3-furanyl)phenyl]-17α-hydroxy-17β-(3-hydroxypropyl)-14β-oestr-4-en-3-one
4'-[17α-hydroxy-17β-(3-hydroxypropyl)-3-oxo-14β-oestr-4-en-11β-yl][1,1'-biphenyl]-4-carbonitrile
(Z)-11β-(4-acetylphenyl)-17α-hydroxy-17β-(3-hydroxy-1-propenyl)-14β-oestr-4-en-3-one
(Z)-4'-[17α-hydroxy-17β-(3-hydroxy-1-propenyl)-3-oxo-14β-oestr-4-en-11β-yl][1,1'-biphenyl]-4-carbonitrile
11β-(4-acetylphenyl)-17α-hydroxy-17β-(methoxymethyl)-14β-oestr-4-en-3-one
11β-(4-acetylphenyl)-17α-hydroxy-3-oxo-14β-oestr-4-ene-17β-acetonitrile
11β-[4-(3-furanyl)phenyl]-17α-hydroxy-17β-(3-hydroxypropyl)-14β-oestra-4,15-dien-3-one
4'-[17α-hydroxy-17β-(3-hydroxypropyl)-3-oxo-14β-oestra-4,15-dien-11β-yl][1,1'-biphenyl]-4-carbonitrile
11β-(4-acetylphenyl)-17α-hydroxy-17β-methyl-14β-oestra-4,15-dien-3-one
17β-hydroxy-11β-(4-methoxyphenyl)-17α-methyloestra-5,14-dien-3-one
11β-[4-(3-furanyl)phenyl]-17β-hydroxy-17α-methyloestra-5,14-dien-3-one
11β-(4-acetylphenyl)-17β-hydroxy-17α-methyloestra-5,14-dien-3-one
11β-(4-acetylphenyl)-17β-hydroxy-17α-(3-hydroxypropyl)oestra-5,14-dien-3-one
4'-[17β-hydroxy-17α-(3-hydroxypropyl)-3-oxo-oestra-4,14-dien-11β-yl][1,1'-biphenyl]-4-carbonitrile
11β-(4-acetylphenyl)-17β-hydroxy-17α-(1-propynyl)oestra-4,14-dien-3-one
11β-(4-acetylphenyl)4',5'-dihydrospiro[oestra-4,14-diene-17β,2'(3'H)-furan]-3-one
11β-[4-(3-furanyl)phenyl]-17β-hydroxy-17α-(3-hydroxypropyl)oestra-4,15-dien-3-one
4'-[17β-hydroxy-17α-(3-hydroxypropyl)-3-oxo-oestra-4,15-dien-11β-yl][1,1'-biphenyl]-4-carbonitrile
11β-[4-(3-furanyl)phenyl]-17β-hydroxy-17α-methyloestra-4,15-dien-3-one
11β-(4-acetylphenyl)-17β-hydroxy-3-oxo-oestra-4,15-diene-17α-acetonitrile
17-hydroxy-17α-methyl-11β-[4-(3-thienyl)phenyl]oestra-4,14-dien-3-one
17β-hydroxy-11β-(4-methoxyphenyl)-17α-methyloestra-4,15-dien-3-one
11β-(4-acetylphenyl)-17β-hydroxy-17α-methyloestra-4,15-dien-3-one
17β-hydroxy-17α-methyl-11β-[4-(3-pyridinyl)phenyl]oestra-4,15-dien-3-one
4'-[17β-hydroxy-17α-methyl-3-oxo-oestra-4,15-dien-11β-yl][1,1'-biphenyl]-4-carbonitrile
4'-[17β-methoxy-17α-methyl-3-oxo-oestra-4,15-dien-11β-yl][1,1'-biphenyl]-4-carbonitrile
17β-hydroxy-11β-(4-methoxyphenyl)-17α-(1-propynyl)oestra-4,15-dien-3-one
4'-[17β-hydroxy-17α-(1-propynyl)-3-oxo-oestra-4,15-dien-11β-yl][1,1'-biphenyl]-4-carbonitrile
11β-[4-(3-furanyl)phenyl]-17β-hydroxy-17α-(1-propynyl)oestra-4,1 5-dien-3-one
11β-(4-acetylphenyl)-17β-hydroxy-17α-(1-propynyl)oestra-4,15-dien-3-one
4'-[4',5'-dihydro-3-oxospiro[oestra-4,15-dien-17β,2'(3'H)-furan]-11β-yl][1,1'-biphenyl]-4-carbonitrile
(Z)-17β-hydroxy-17α-(3-hydroxy-1-propenyl)-11β-(4-methoxyphenyl)oestra-4,15-dien-3-one
11β-(4-acetylphenyl)-17α-ethynyl-17β-hydroxyoestra-4,15-dien-3-one
4'-[17α-ethynyl-17β-hydroxy-3-oxo-oestra-4,15-dien-11β-yl][1,1'-biphenyl]-4-carbonitrile
11β-[4-dimethylamino)phenyl]-17β-hydroxy-17α-methyloestra-4,15-dien-3-one.

3. Process for the preparation of compounds of the general formula I 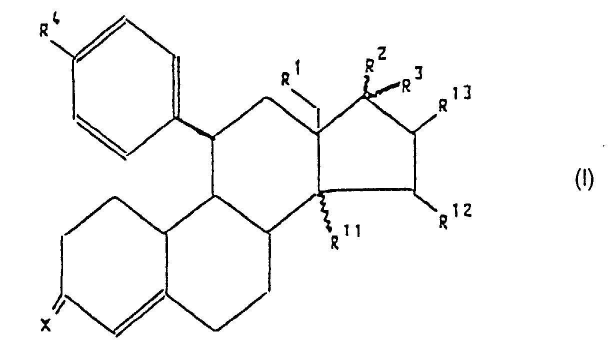 wherein X, R¹, R², R³, R⁴, R¹¹, R¹² and R¹³ are as defined in claim 1, characterised in that a compound of the general formula II 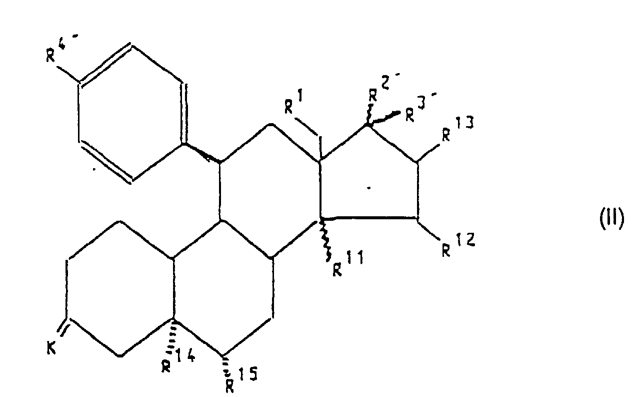 wherein K is a keto group protected in the form of ketal, R¹, R¹¹, R¹² and R¹³ are as defined for formula I and R^{2'}, R^{3'} and R^{4'} are as defined for R², R³ and R⁴, respectively, in formula I with the exception of the cyanide radical for R⁴, any hydroxy, mercapto, amino, oxo and/or terminal acetylene groups that are present optionally being protected,
and R¹⁴ represents a hydroxy group and R¹⁵ represents hydrogen, or R¹⁴ and R¹⁵ together form a double bond,
is subjected in known manner to the action of an acid agent in order to free the 3-oxo group and also the other protected groups and, where appropriate, to remove water, and then, if desired, any hydroxy, mercapto and/or amino group(s) present in R² and/or R³ and/or R⁴ are alkylated or acylated, if desired a cyanide radical is introduced into the 11β-aryl substituent, if desired the amino or sulphide group that may be contained in R⁴ is oxidised, if desired reaction is carried out with hydroxylamine hydrochloride to form the product of the general formula I wherein X represents the hydroxyimino grouping 〉N∼OH, or the 3-oxo group is converted into a product of the general formula I wherein X represents 2 hydrogen atoms, and optionally a pharmaceutically tolerable acid addition salt is prepared.

4. Pharmaceutical preparations that comprise at least one compound according to claim 1 or claim 2 and also a pharmaceutical carrier.

5. Use of the compounds according to claim 1 or claim 2 in the manufacture of medicaments.

## Revendications

1. Composés de formule générale I 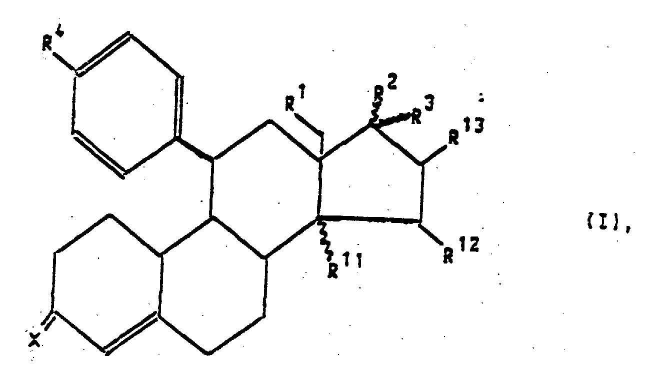 dans laquelle, soit
la) R¹¹ représente un atome d'hydrogène en position β ainsi que R¹² et R¹³ chacun représente un atome d'hydrogène, soit
Ib) R¹¹ représente un atome d'hydrogène en position β ainsi que R¹² et R¹³ ensemble représentent une deuxième liaison, soit
Ic) R¹¹ et R¹² ensemble représentent une deuxième liaison, ainsi que R¹³ un atome d'hydrogène, soit
Id) R¹¹ représente un atome d'hydrogène en position α ainsi que R¹² et R¹³ représentent une deuxième liaison,
ainsi que Ia), Ib), Ic) ou Id)
X représente un atome d'oxygène, le groupement hydroxyimino >N∼OH ou deux atomes d'hydrogène,
R¹ représente un atome d'hydrogène ou un groupe méthyle,
R² représente un groupe hydroxy, un groupe alcoxy en C₁-C₁₀ ou acyloxy en C₁-C₁₀,
R³ représente un atome d'hydrogène, le groupement -(CH₂)ₙCH₂Z, n étant 0, 1, 2, 3, 4 ou 5, Z représente un atome d'hydrogène, le groupe cyano ou le reste -OR⁵ avec R⁵ = H, alkyle en C₁-C₁₀ ou acyle en C₁-C₁₀, le groupement -(CH₂)ₘ-C≡C-Y, m étant 0, 1 ou 2 et Y un atome d'hydrogène, de fluor, de chlore, de brome ou d'iode, un reste hydroxyalkyle en C₁-C₁₀, alcoxyalkyle en C₁-C₁₀, acyloxyalkyle en C₁-C₁₀, ou lorsque m = 0, représentent un groupe méthyle, en plus le groupement -(CH₂)ₚ-CH=CH-(CH₂)ₖCH₂R⁶, p valant 0 ou 1 et k valant 0, 1 ou 2, et R⁶ représente un atome d'hydrogène, un groupe hydroxy, un reste alcoxy en C₁-C₄ ou acyloxy en C₁-C₄,
dans Ia) et Ib), R² étant en position α et R³ en position β et
dans Ic) et Id) R² étant en position β et R³ en position α,
ou bien R² et R³ ensemble représentent un reste de formule 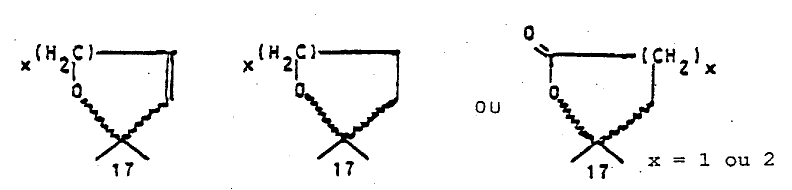
R⁴ étant un atome d'hydrogène, un groupe cyano, un atome de chlore,d e fluor, de brome, d'iode, un groupe trialkylsilyle, trialkylstannyle, un reste alcoxyalkyle, alkylacyle ou alkyle en C₁-C₈, un groupe amino 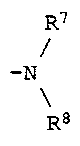 dans laquelle R⁷ et R⁸,
indépendamment l'un de l'autre, sont un atome d'hydrogène ou un groupe alkyle en C₁-C₄ ou un aminoxyde correspondant 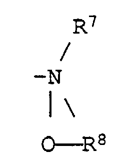 ou représente les groupements -OR⁹ ou -S(O)ᵢR⁹ avec i = 0, 1 ou 2, dans lesquels R⁹ représente un atome d'hydrogène, un groupe méthyle, éthyle, propyle, isopropyle, méthoxyphényle, allyle ou 2-diméthylaminoéthyle ou un reste hétéroaryle de formule Iα 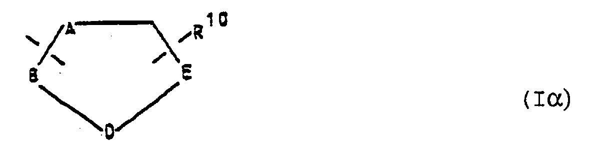 dans laquelle A est un atome d'azote, d'oxygène ou de soufre, -B-D-E- représente la succession d'éléments -C-C-C-, -N-C-C- ou -C-N-C- et R¹⁰ un atome d'hydrogène, un groupe cyano, un atome de chlore, de fluor, de brome, d'iode, un groupe trialkylsilyle, trialkylstannyle, un reste alcoxyalkyle, alkylacyle ou alkyle en C₁-C₈, un groupe amino 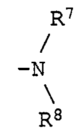 dans lequel R⁷ et R⁸, indépendamment l'un de l'autre représentent un atome d'hydrogène ou un groupe alkyle en C₁-C₄, ou un aminoxyde correspondant 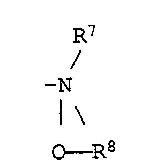 ou le groupement -OR⁹ ou S(O)ᵢR⁹ avec i = 0, 1 ou 2, dans lesquels R⁹ représente un atome d'hydrogène, un groupe méthyle, éthyle, propyle, isopropyle, méthoxyphényle, allyle ou 2-diméthylaminoéthyle,
ou représenstent un reste hétéroaryle de formule Iβ 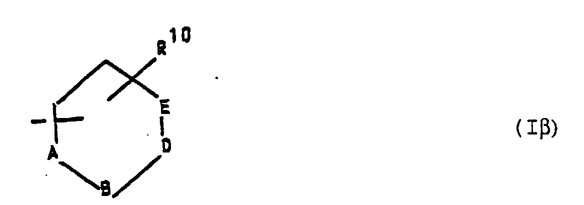 dans laquelle A est un atome d'azote et -B-D-E- représente la succession d'éléments -C-C-C-, -N-C-C-, -C-C-N- et R¹⁰ a la signification déjà donnée,
ou représente un reste phényle de formule Iγ 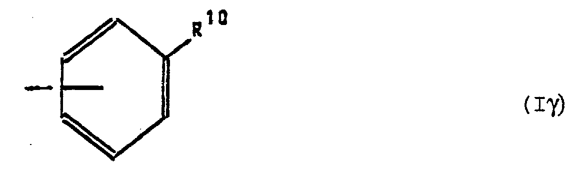 dans laquelle R¹⁰ a la signification déjà donnée,
ainsi que leurs sels d'addition avec des acides pharmacologiquement tolérés.

2. Composés selon la revendication 1, notamment 17α-hydroxy-17β-(3-hydroxypropyl)-11β-[4-(3-pyridyl)-phényl]-14β-estr-4-èn-3-one
11β-(4-acétylphényl)-17α-hydroxy-17β-(3-hydroxypropyl)-14β-estr-4-èn-3-one
11β-[4-(3-furanyl)phényl]-17α-hydroxy-17β-(3-hydroxypropyl)-14β-estr-4-èn-3-one
4'-[17α-hydroxy-17β-(3-hydroxypropyl)-3-oxo-14β-estr-4-èn-11β-yl][1,1'-biphényl]-4-carbonitrile
(Z)-11β-(4-acétylphényl)-17α-hydroxy-17β-(3-hydroxy-1-propényl)-14β-estr-4-èn-3-one
(Z)-4'-[17α-hydroxy-17β-(3-hydroxy-1-propényl)-3-oxo-14β-estr-4-èn-11β-yl][1,1'-biphényl]-4-carbonitrile
11β-(4-acétylphényl)-17α-hydroxy-17β-(3-méthoxyméthyl)-14β-estra-4-èn-3-one
11β-(4-acétylphényl)-17α-hydroxy-3-oxo-14β-estra-4-èn-17β-acétonitrile
11β-[4-(3-furanyl)phényl]-17α-hydroxy-17β-(3-hydroxypropyl)-14β-estra-4,15-dièn-3-one
4'-[17α-hydroxy-17β-(3-hydroxypropyl)-3-oxo-14β-estr-4,15-dièn-11β-yl][1,1'-biphényl]-4-carbonitrile
11β-(4-acétylphényl)-17α-hydroxy-17β-méthyl-14β-estr-4,15-dièn-3-one
17β-hydroxy-11β-(4-méthoxyphényl)-17α-méthylestra-5,14-dièn-3-one
11β-[4-(3-furanyl)phényl]-17β-hydroxy-17β-méthylestra-5,14-dièn-3-one
11β-(4-acétylphényl)-17β-hydroxy-17α-méthylestra-5,14-dièn-3-one
11β-(4-acétylphényl)-17β-hydroxy-17α-(3-hydroxypropyl)-estra-5,14-dièn-3-one
4'-[17β-hydroxy-17α-(3-hydroxypropyl)-3-oxoestra-4,14-dièn-11β-yl][1,1'-biphényl]-4-carbonitrile
11β-(4-acétylphényl)-17β-hydroxy-17α-(1-propynyl)estra-4,14-dièn-3-one
11β-(4-acétylphényl)-4',5'-dihydrospira[estra-4,14-dièn-17β,2'(3'H)-furan]-3-one
11β-[4-(3-furanyl)phényl]-17β-hydroxy-17α-(3-hydroxypropyl)estra-4,15-dièn-3-one
4'-[17β-hydroxy-17α-(3-hydroxypropyl)-3-oxoestra-4,15-dièn-11β-yl][1,1'-biphényl]-4-carbonitrile
11β-[4-(3-furanyl)phényl]-17β-hydroxy-17α-méthylestra-4,15-dièn-3-one
11β-(4-acétylphényl)-17β-hydroxy-3-oxoestra-4,15-dièn-17αacétonitrile
17-hydroxy-17α-méthyl-11β-[4-(3-thiényl)phényl]estra-4,14-dièn-3-one
17β-hydroxy-11β-(4-méthoxyphényl)-17α-méthylestra-4,15-dièn-3-one
11β-(4-acétylphényl)-17β-hydroxy-17α-méthylestra-4,15-dièn-3-one
17β-hydroxy-17α-méthyl-11β-[4-(3-pyridyl)phényl]estra-4,15-dièn-3-one
4'-[17β-hydroxy-17α-méthyl-3-oxoestra-4,15-dièn-11β-yl]-[1,1'-biphényl]-4-carbonitrile
4'-[17β-méthoxy-17α-méthyl-3-oxoestra-4,15-dièn-11β-yl]-[1,1'-biphényl]-4-carbonitrile.
17β-hydroxy-11β-(4-méthoxyphényl)-17α-(1-propynyl)estra-4,15-dièn-3-one
4'-[17β-hydroxy-17α-(1-propynyl)-3-oxoestra-4,15-dièn-11β-yl]-[1,1'-biphényl]-4-carbonitrile
11β-[4-(3-furanyl)phényl]-17β-hydroxy-17α-(1-propynyl)-estra-4,15-dièn-3-one
11β-(4-acétylphényl)-17β-hydroxy-17α-(1-propynyl)-estra-4,15-dièn-3-one
4'[4',5'-dihydro-3-oxaspiro[estra-4,15-dièn-17β,2'(3'H)-furan]-11β-yl][1,1'-biphényl]-4-carbonitrile
(Z)-17β-hydroxy-17α-3-hydroxy-(1-propényl)-11β-(4-méthoxyphényl)estra-4,15-dièn-3-one
11β-(4-acétylphényl)-17α-(éthynyl)-17β-hydroxyestra-4,15-dièn-3-one
4'-[17α-éthynyl-17β-hydroxy-3-oxoestra-4,15-dièn-11β-yl][1,1'-biphényl]-4-carbonitrile
11β-[4-(diméthylamino)phényl]-17β-hydroxy-17α-méthylestra-4,15-dièn-3-one

3. Procédé pour la préparation de composés de formule générale I 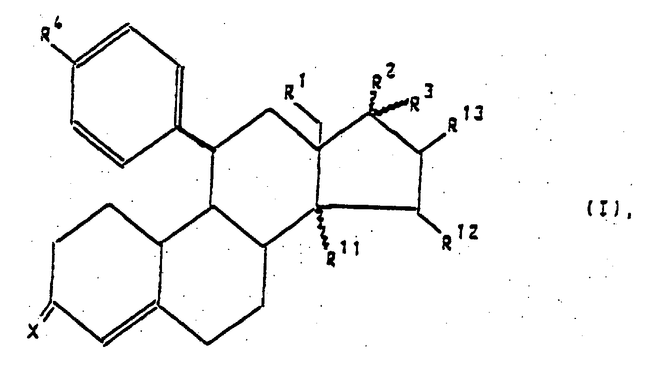 dans laquelle X, R¹, R², R³, R⁴, R¹¹, R¹² et R¹³ ont la signification donnée dans la revendication 1, caractérisé en ce que l'on soumet un composé de formule générale II 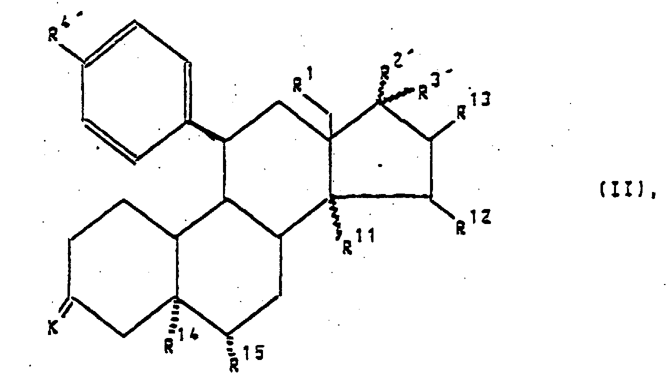 dans laquelle K est un groupe céto protégé sous forme de cétal, R¹, R¹¹, R¹² et R¹³ ont la même signification que dans la formule I ainsi que R², R³ et R⁴, en excluant le reste cyanure pour R⁴, ont la même signification que R², R³, respectivement R⁴ dans la formule I, dans laquelle les groupes hydroxy, mercapto, amino, oxo et/ou groupes terminaux acétylèno sont éventuellement protégés, ainsi que R¹⁴ est un groupe hydroxy et R¹⁵ un hydrogène ou R¹⁴ et R¹⁵ ensemble représentent une double liaison, de façon connue en soi, à l'action d'un agent acide pour libérer le groupe 3-oxo ainsi que les autres groupes protégés et éventuellement pour dissocier l'eau, et ensuite, si on le souhaite, on effectue une alkylation, respectivement acylation des groupes hydroxy, mercapto et/ou amino présents dans R² et/ou R³ et/ou R⁴, si on le souhaite, on introduit un reste cyanure dans le substituant 11β-aryle, si on le souhaite, on oxyde le groupe amino ou sulfure éventuellement contenu dans R⁴, si on le souhaite on fait réagir avec le chlorhydrate d'hydroxylamine pour obtenir un produit de formule générale I avec X ayant la signification d'un groupement hydroxyimino >N∼O ou on transforme le groupe 3-oxo en un produit de formule générale I, dans laquelle X représente 2 atomes d'hydrogène, ainsi qu'on prépare éventuellement un/des sel(s) d'addition d'acide pharmaceutiquement toléré(s).

4. Préparations pharmaceutiques qui comportent au moins un composé selon la revendication 1 ou 2, ainsi qu'un support pharmaceutique.

5. Utilisation des composés selon la revendication 1 ou 2, pour la préparation de médicaments.
